# EUROPEAN PATENT APPLICATION

(11) **EP 4 082 575 A1**
(43) Date of publication of application: **02.11.2022**
(21) Application number: 20906713.1
(22) Date of filing: 25.12.2020
(51) Int. Cl.: A61K 45/00, A61P 35/00, A61P 43/00, C12N 5/09, C12Q 1/02, C12Q 1/68, C12N 9/99, G01N 33/15, G01N 33/50, G01N 33/574, A61K 31/437, A61K 31/496

(54) **METHOD FOR PREDICTING SENSITIVITY OF CANCER CELL TO GPX4 INHIBITOR**

(30) Priority: 26.12.2019 JP 2019236516
(71) Applicant: CHUGAI SEIYAKU KABUSHIKI KAISHA, Tokyo 115-8543 (JP)
(72) Inventor: OGASAWARA Kiyomoto, Kamakura-shi, Kanagawa 247-8530 (JP); HASHIMOTO Keisuke, Kamakura-shi, Kanagawa 247-8530 (JP); KASHIMA Kenji, Kamakura-shi, Kanagawa 247-8530 (JP); SAKAMOTO Hiroshi, Kamakura-shi, Kanagawa 247-8530 (JP)
(74) Representative: Vossius & Partner Patentanwälte Rechtsanwälte mbB
(86) International application number: PCT/JP2020/048811
(87) International publication number: WO 2021/132592

(57) **Abstract**

Provided are a cancer therapeutic drug comprising a compound which inhibits GPX4 as an active ingredient, the cancer therapeutic drug treating cancer containing a cancer cell having a suppressed function of a SWI/SNF complex factor detected; and a method for predicting sensitivity of a cancer cell to a GPX4 inhibitor, the method comprising the step of predicting a cancer cell having a suppressed function of a SWI/SNF complex factor detected in the cancer cell, as having sensitivity to the GPX4 inhibitor.

## Description

### [Technical Field]

The present invention relates to a method for predicting sensitivity of a cancer cell to a GPX4 inhibitor. The present invention also relates to a method for predicting sensitivity of a cancer patient to treatment with a GPX4 inhibitor, a method for selecting a cancer patient for cancer treatment with a GPX4 inhibitor, a method for suppressing growth of cancer cells, a method for treating cancer, a method for screening compounds to be used for cancer treatment, and a cancer therapeutic drug.

### [Background Art]

In recent years, due to rapid advancement of genome sequence techniques, it has been possible to analyze the genome information including unique gene mutations in cancer cells. For development of anticancer drugs, inhibitors have been discovered which inhibit the functions specifically of a cancer cell having a gain-of-function gene mutation typified by EGFR gene mutation, BRAF gene mutation, an ALK fusion gene, a ROS1 fusion gene or the like (Non Patent Literatures 1 to 3 etc.). Treatment methods which target a cancer cell having such a gene mutation and are specific to the cancer cell are expected as treatment methods with high cancer selectivity and a high efficacy.

On the other hand, gene mutations found in human cancer cells include not only the gain-of-function gene mutations but also the loss-of-function gene mutations. For a loss-of-function gene mutation, it is difficult to discover a drug specific to the gene mutation, and a treatment strategy different from treatment targeting a cancer cell having a gain-of-function gene mutation is required.

The SWI/SNF complex is a chromatin remodeling factor including 12 to 15 subunits (complex factors). In recent years, suppressed functions such as loss-of-function gene mutations and suppressed expression of SWI/SNF complex factors have been reported to be observed in many human cancers, and have been pointed as being involved in tumor development and advancement. Since these mutations are thought to suppress important functions of the SWI/SNF complex such as dissociation of DNA stored in nucleosome and recruiting of transcription factors and transcription modulating factors such as histone deacetylases in DNA, there may be an important relationship between such a suppressed function and cancer development and advancement, and studies have been conducted.

For Example, Non Patent Literature 4 describes that in non-small cell lung cancer, deficiency of SMARCA4 which is a SWI/SNF complex factor is in a synthetic lethal relationship with a CDK 4/6 inhibitor, Non Patent Literature 5 describes that lung cancer deficient in SMARCA4 had increased sensitivity to an OXPHOS (mitochondrial oxidative phosphorylation) inhibitor, Non Patent Literature 6 describes that the mutation of a gene for SMARCA4 in non-small cell lung cancer had increased sensitivity to an Aurora kinase inhibitor, and Non Patent Literature 7 describes that the mutation of a gene for ARID1A which is likewise a SWI/SNF complex factor is in a synthetic lethal relationship with a GSH synthesis inhibitor.

However, the treatment strategy specifically targeting a cancer cell having a suppressed function of a SWI/SNF complex has not been sufficient yet.

### [Citation List]

### [Non Patent Literature]

[Non Patent Literature 1] Makoto Maemondo et al., NEJM 2010 Jun 24; 362 (25), p. 2380-2388
[Non Patent Literature 2] Paul B. Chapman et al., NEJM 2011 Jun 30; 364 (26), p. 2507-2516
[Non Patent Literature 3] D. Ross Camidge et al., J Thorac Oncol. 2019 Jul; 14 (7), p. 1233-1243
[Non Patent Literature 4] Yibo Xue et al., Nat. Commun. 10: 557, 2019
[Non Patent Literature 5] Yonathan Lissanu Deribe et al., Nat. Med. Vol 24, July 2018, p. 1047-1057
[Non Patent Literature 6] Vural Tagal et al., Nat. Commun. 8: 14098, 2017
[Non Patent Literature 7] Hideaki Ogiwara et al., 2019, Cancer Cell 35, p. 177-190

### [Summary of Invention]

### [Technical Problem]

The present invention has been made in view of the circumstances described above. An object of the present invention is to develop a treatment strategy capable of specifically targeting a cancer cell having a suppressed function of a SWI/SNF complex. More specifically, the object of the present invention is to develop a treatment strategy specifically targeting a cancer cell having a suppressed function of a SWI/SNF complex factor detected.

### [Solution to Problem]

The present inventors have extensively conducted studies for solving the above-described problems, and resultantly found that when expression of GPX4 is suppressed or the function of GPX4 is inhibited in a cancer cell having a suppressed function of a SWI/SNF complex factor detected, growth of the cancer cells is markedly suppressed and/or cell death is induced, whereas such suppression of growth and/or cell death do not occur in a cell which does not have a suppressed function of a SWI/SNF complex factor.

More specifically, the present inventors have conducted exhaustive knockdown experiments on human cancer cell lines, to extensively investigate which drug suppressing a function of a protein leads to antitumor activity against a human cancer cell having a suppressed function of a SWI/SNF complex factor. As a result, it has been found that when expression of GPX4 is suppressed in a cancer cell line having a deficiency type mutation in a gene for SMARCA4 that is a core component of a SWI/SNF complex and/or having no expressed SMARCA4 protein detected, cell death of the cancer cell is induced to markedly suppress cell growth.

The present inventors have found that even when the activity of GPX4 is inhibited using a compound reported to inhibit the enzymatic activity of GPX4 (e.g. ML210 or RSL3), cell death of a cancer cell having a suppressed function of SMARCA4 is induced to markedly suppress cell growth as in the case where expression of GPX4 is suppressed.

Further, as molecules belonging to a GPX family in a human, eight types of molecules of GPX1, GPX2, GPX3, GPX4, GPX5, GPX6, GPX7 and GPX8 have been reported, and the present inventors have found that in particular, inhibition of GPX4, among the molecules, specifically exhibits antitumor activity against a cancer cell having a suppressed function of SMARCA4.

On the other hand, since SMARCA4 is a protein forming a SWI/SNF complex, it has been thought that GPX4 may also be effective on a cell having a suppressed function of another protein forming a SWI/SNF complex. Thus, antitumor activity by suppression of expression and/or inhibition of activity of GPX4 has been evaluated using a cancer cell line having a suppressed function in other SWI/SNF complex factors in addition to SMARCA4 (e.g. SMARCA2, ARID1A, ARID1B, ARID2 and BCL11B), and resultantly, it has been found that even in a cancer cell line having a suppressed function in other SWI/SNF complex factors, suppression of cell growth and cell death are markedly induced by inhibition of GPX4 as in a cancer cell line having a suppressed function of SMARCA4.

Since GPX4 is an enzyme which consumes glutathione in cells to perform hydrolysis, it has been thought that inhibition of a group of other factors (proteins) involved in glutathione synthesis may exhibit antitumor activity against a cancer cell having a suppressed function of a SWI/SNF complex factor. However, it has been revealed that even when a group of other factors related to glutathione synthesis (e.g. gamma-glutamylcysteine synthetase(GCLC), glutathione synthetase(GSS), Glutamate-Cysteine Ligase Modifier Subunit(GCLM), Microsomal Glutathione S-Transferase 1 (MGST1), Microsomal Glutathione S-Transferase 3 (MGST3), Glutathione-Disulfide Reductase (GSR) and glucose-6-phosphate dehydrogenase (G6PD)) are inhibited, sufficient antitumor activity cannot be obtained as compared to inhibition of GPX4, and it is particularly important to inhibit GPX4 for obtaining antitumor activity targeting a cancer cell having a suppressed function of a SWI/SNF complex factor.

Further, the present inventors have demonstrated that in a tumor-bearing mouse into which a cancer cell having a suppressed function of SWI/SNF complex factor is transplanted, an increase in tumor volume is markedly suppressed by administration of a compound (GPX4 inhibitor) reported to inhibit enzymatic activity of GPX4, and inhibition of GPX4 reliably exhibits an antitumor effect even *in vivo.*

Thus, the present inventors have found that treatment for inhibiting GPX4 (suppression of expression or inhibition of activity) is a promising approach for treatment targeting a cancer cell having a suppressed function of a SWI/SNF complex factor. It has also been revealed that in this treatment strategy, it is possible to select a cancer patient using a suppressed function of a SWI/SNF complex factor as an indicator, followed by administering a GPX4 inhibitor, so that efficient treatment based on companion diagnosis is possible.

Further, the present inventors have also found that screening of drugs useful for treatment of cancer having a suppressed function of a SWI/SNF complex factor can be performed on the basis of whether GPX4 is inhibited or not, leading to completion of the present invention.

Accordingly, the present invention relates to a treatment method specifically targeting a cancer cell having a suppressed function of a SWI/SNF complex factor, and companion diagnosis for the treatment method. More specifically, the present invention provides the following.
[1] A cancer therapeutic drug comprising a compound which inhibits GPX4 as an active ingredient, the cancer therapeutic drug being a therapeutic drug for cancer containing a cancer cell having a suppressed function of a SWI/SNF complex factor detected.
[2] A cancer therapeutic drug comprising a compound which inhibits GPX4 as an active ingredient, the cancer therapeutic drug being a therapeutic drug for treating a cancer patient having a suppressed function of a SWI/SNF complex factor detected in a cancer cell contained in a cancer patient-derived sample.
[3] A cancer therapeutic drug comprising a compound, which inhibits GPX4, as an active ingredient, the cancer therapeutic drug being a therapeutic drug to be administered to a cancer patient having a suppressed function of a SWI/SNF complex factor detected in a cancer cell contained in a cancer patient-derived sample.
[4] A method for predicting sensitivity of a cancer cell to a GPX4 inhibitor, the method comprising the step of:
   predicting a cancer cell having a suppressed function of a SWI/SNF complex factor detected in the cancer cell, as having sensitivity to a GPX4 inhibitor.
[5] A method for predicting sensitivity of a cancer cell to a GPX4 inhibitor, the method comprising the steps of:
   (a) detecting the presence or absence of a suppressed function of a SWI/SNF complex factor in the cancer cell; and
   (b) predicting a cancer cell having the suppressed function of a SWI/SNF complex factor, as having sensitivity to a GPX4 inhibitor.
[6] A method for predicting sensitivity of a cancer patient to treatment with a GPX4 inhibitor, the method comprising the step of:
   predicting a cancer patient having a suppressed function of a SWI/SNF complex factor detected in a cancer cell contained in a cancer patient-derived sample, as having sensitivity to treatment with a GPX4 inhibitor.
[7] A method for predicting sensitivity of a cancer patient to treatment with a GPX4 inhibitor, the method comprising the steps of:
   (a) detecting the presence or absence of a suppressed function of a SWI/SNF complex factor in a cancer cell contained in a cancer patient-derived sample; and
   (b) predicting a cancer patient having the suppressed function of a SWI/SNF complex factor detected in the cancer cell, as having sensitivity to treatment with a GPX4 inhibitor.
[8] A method for selecting a cancer patient for cancer treatment with a GPX4 inhibitor, the method comprising the step of:
   selecting a cancer patient having a suppressed function of a SWI/SNF complex factor detected in a cancer cell contained in a cancer patient-derived sample, for cancer treatment with a GPX4 inhibitor.
[9] A method for selecting a cancer patient for cancer treatment with a GPX4 inhibitor, the method comprising the steps of:
   (a) detecting the presence or absence of a suppressed function of a SWI/SNF complex factor in a cancer cell contained in a cancer patient-derived sample; and
   (b) selecting a cancer patient having the suppressed function of a SWI/SNF complex factor detected in the cancer cell, for cancer treatment with a GPX4 inhibitor.
[10] A method for suppressing growth of cancer cells having a suppressed function of a SWI/SNF complex factor detected, the method comprising the step of:
   contacting a GPX4 inhibitor with a cancer cell having the suppressed function of a SWI/SNF complex factor detected.
[11] A method for treating cancer, the method comprising the step of:
   administering a GPX4 inhibitor to a cancer patient having a suppressed function of a SWI/SNF complex factor detected in a cancer cell contained in a cancer patient-derived sample.
[12] A method for treating cancer, the method comprising the steps of:
   (a) detecting the presence or absence of a suppressed function of a SWI/SNF complex factor in a cancer cell contained in a cancer patient-derived sample; and
   (b) administering a GPX4 inhibitor to a cancer patient having the suppressed function of a SWI/SNF complex factor detected in the cancer cell.
[13] A method for screening compounds to be used for treatment of cancer containing a cancer cell having a suppressed function of a SWI/SNF complex factor detected, the method comprising the step of:
   selecting a compound on the basis of whether GPX4 is inhibited or not.
[14] The cancer therapeutic drug according to any one of [1] to [3], wherein the SWI/SNF complex factor is a BAF complex factor.
[15] The method according to any one of [4] to [13], wherein the SWI/SNF complex factor is a BAF complex factor.
[16] The cancer therapeutic drug according to any one of [1] to [3], wherein the SWI/SNF complex factor is at least one selected from the group consisting of SMARCA2, SMARCA4, ARID1A, ARID1B, ARID2 and BCL11B.
[17] The method according to any one of [4] to [13], wherein the SWI/SNF complex factor is at least one selected from the group consisting of SMARCA2, SMARCA4, ARID1A, ARID1B, ARID2 and BCL11B.
[18] The cancer therapeutic drug according to any one of [1] to [3], wherein the suppressed function of a SWI/SNF complex factor is a decrease in activity of a SWI/SNF complex having the SWI/SNF complex factor as a constituent factor and/or a decrease in expression of the SWI/SNF complex factor.
[19] The method according to any one of [4] to [13], wherein the suppressed function of a SWI/SNF complex factor is a decrease in activity of a SWI/SNF complex having the SWI/SNF complex factor as a constituent factor and/or a decrease in expression of the SWI/SNF complex factor.
[20] The cancer therapeutic drug according to any one of [1] to [3], wherein the suppressed function of a SWI/SNF complex factor is a loss-of-function mutation of a gene for the SWI/SNF complex factor.
[21] The method according to any one of [4] to [13], wherein the suppressed function of a SWI/SNF complex factor is a loss-of-function mutation of a gene for the SWI/SNF complex factor.
[22] A cancer therapeutic drug comprising a compound, which inhibits GPX4, as an active ingredient, the cancer therapeutic drug being a therapeutic drug for cancer containing a cancer cell having a loss-of-function mutation of a gene for a SWI/SNF complex factor detected.
[23] A cancer therapeutic drug comprising a compound, which inhibits GPX4, as an active ingredient, the cancer therapeutic drug being a therapeutic drug for treating a cancer patient having a loss-of-function mutation of a gene for a SWI/SNF complex factor detected in a cancer cell contained in a cancer patient-derived sample.
[24] A cancer therapeutic drug comprising a compound which inhibits GPX4 as an active ingredient, the cancer therapeutic drug being a therapeutic drug to be administered to a cancer patient having a loss-of-function mutation of a gene for a SWI/SNF complex factor detected in a cancer cell contained in a cancer patient-derived sample.
[25] A method for predicting sensitivity of a cancer cell to a GPX4 inhibitor, the method comprising the steps of:
   predicting a cancer cell having a loss-of-function mutation of a gene for a SWI/SNF complex factor detected in the cancer cell, as having sensitivity to a GPX4 inhibitor.
[26] A method for predicting sensitivity of a cancer cell to a GPX4 inhibitor, the method comprising the steps of:
   (a) detecting the presence or absence of a loss-of-function mutation of a gene for a SWI/SNF complex factor in the cancer cell; and
   (b) predicting a cancer cell having the loss-of-function mutation of a gene for a SWI/SNF complex factor detected, as having sensitivity to a GPX4 inhibitor.
[27] A method for predicting sensitivity of a cancer patient to treatment with a GPX4 inhibitor, the method comprising the step of:
   predicting a cancer patient having a loss-of-function mutation of a gene for a SWI/SNF complex factor detected in a cancer cell contained in a cancer patient-derived sample, as having sensitivity to treatment with a GPX4 inhibitor.
[28] A method for predicting sensitivity of a cancer patient to treatment with a GPX4 inhibitor, the method comprising the steps of:
   (a) detecting the presence or absence of a loss-of-function mutation of a gene for a SWI/SNF complex factor in a cancer cell contained in a cancer patient-derived sample; and
   (b) predicting a cancer patient having the loss-of-function mutation of a gene for a SWI/SNF complex factor detected in the cancer cell, as having sensitivity to treatment with a GPX4 inhibitor.
[29] A method for selecting a cancer patient for cancer treatment with a GPX4 inhibitor, the method comprising the step of:
   selecting a cancer patient having a loss-of-function mutation of a gene for a SWI/SNF complex factor detected in a cancer cell contained in a cancer patient-derived sample, for cancer treatment with a GPX4 inhibitor.
[30] A method for selecting a cancer patient for cancer treatment with a GPX4 inhibitor, the method comprising the steps of:
   (a) detecting the presence or absence of a loss-of-function mutation of a gene for a SWI/SNF complex factor in a cancer cell contained in a cancer patient-derived sample; and
   (b) selecting a cancer patient having the loss-of-function mutation of a gene for a SWI/SNF complex factor detected in the cancer cell, for cancer treatment with a GPX4 inhibitor.
[31] A method for suppressing growth of cancer cells having a loss-of-function mutation of a gene for a SWI/SNF complex factor detected, the method comprising the step of:
   contacting a GPX4 inhibitor with a cancer cell having the loss-of-function mutation of a gene for a SWI/SNF complex factor detected.
[32] A method for treating cancer, the method comprising the step of:
   administering a GPX4 inhibitor to a cancer patient having a loss-of-function mutation of a gene for a SWI/SNF complex factor detected in a cancer cell contained in a cancer patient-derived sample.
[33] A method for treating cancer, the method comprising the steps of:
   (a) detecting the presence or absence of a loss-of-function mutation of a gene for a SWI/SNF complex factor in a cancer cell contained in a cancer patient-derived sample; and
   (b) administering a GPX4 inhibitor to a cancer patient having the loss-of-function mutation of a gene for a SWI/SNF complex factor detected in the cancer cell.
[34] A method for screening compounds to be used for treatment of cancer containing a cancer cell having a loss-of-function mutation of a gene for a SWI/SNF complex factor detected, the method comprising the step of:
   selecting a compound on the basis of whether GPX4 is inhibited or not.
[35] The cancer therapeutic drug according to any one of [22] to [24], wherein the SWI/SNF complex factor is a BAF complex factor.
[36] The cancer therapeutic drug according to any one of [25] to [34], wherein the SWI/SNF complex factor is a BAF complex factor.
[37] The cancer therapeutic drug according to any one of [22] to [24], wherein the SWI/SNF complex factor is at least one selected from the group consisting of SMARCA2, SMARCA4, ARID1A, ARID1B, ARID2 and BCL11B.
[38] The method according to any one of [25] to [34], wherein the SWI/SNF complex factor is at least one selected from the group consisting of SMARCA2, SMARCA4, ARID1A, ARID1B, ARID2 and BCL11B.

### [Advantageous Effects of Invention]

According to the present invention, it is possible to efficiently predict sensitivity to cancer treatment with a GPX4 inhibitor using a suppressed function of a SWI/SNF complex factor (e.g. gene mutation or a decrease in expression of protein in SWI/SNF complex factors typified by SMARCA4 and ARID1A) as an indicator. In addition, according to the present invention, it is possible to detect the presence or absence of a suppressed function of a SWI/SNF complex factor in a cancer patient-derived sample and select a patient having the mutation detected, followed by subjecting the patient to treatment of cancer with a GPX4 inhibitor. This enables significant improvement of cancer treatment outcomes. By using a probe or a primer against a gene for a SWI/SNF complex factor and an antibody against a SWI/SNF complex factor, companion diagnosis can be efficiently performed by detection of the presence or absence of such a suppressed function of the SWI/SNF complex factor.

### [Brief Description of Drawings]

[Figure 1] Figure 1 is a graph showing cell growth inhibition ratios (CGI (%)) in suppression of expression of GPX4 (#1 and #2) in the cell lines of NCI-H1792, MOR, NCI-H2110, NCI-H522, NCI-H23 and SNU-1327.
[Figure 2] Figure 2 is a graph showing cell growth inhibition ratios (CGI (%)) in suppression of expression of GPX4 (#2) in the cell lines shown in Table 4.
[Figure 3] Figure 3 is a graph showing cell growth inhibition ratios (CGI (%)) in suppression of expression of GPX4 (#3) in the cell lines shown in Table 4.
[Figure 4] Figure 4 is a graph showing IC50 (µM) in the cell lines shown in Table 4 when ML210 is used as a GPX4 inhibitor.
[Figure 5] Figure 5 is a graph showing IC50 (µM) in the cell lines shown in Table 4 when RSL3 is used as a GPX4 inhibitor.
[Figure 6] Figure 6 is a graph showing cell growth inhibition ratios (CGI (%)) in suppression of expression of GPX family proteins in the cell lines of NCI-H2110, NCI-H522 and NCI-H23.
[Figure 7] Figure 7 is a graph showing cell growth inhibition ratios (CGI (%)) in suppression of expression of proteins involved in glutathione synthesis in the cell lines of NCI-H2110, NCI-H522 and NCI-H23.
[Figure 8] Figure 8 is a graph showing IC50 (µM) in the cell lines MOR, NCI-H358, NCI-H23 and NCI-H522 when ML210, RSL3 or BSO is used.
[Figure 9] Figure 9 is a graph showing a relationship between the volume of a tumor and the time after inoculation of SK-HEP-1 in each group of SK-HEP-1 tumor-bearing mice (ML210 or vehicle).

### [Description of Embodiments]

Hereinafter, the present invention will be described in detail along preferred embodiments thereof.

### <Method for predicting sensitivity of cancer cell to GPX4 inhibitor, method for predicting sensitivity of patient to treatment with GPX4 inhibitor, and method for selecting a cancer patient for cancer treatment with GPX4 inhibitor>

In the present invention, it has been found that when GPX4 is inhibited in a cancer cell having a suppressed function of a SWI/SNF complex factor, growth of the cancer cell can be suppressed. According to this finding, sensitivity of a cancer cell to a GPX4 inhibitor can be predicted using a suppressed function of a SWI/SNF complex factor as an indicator. Accordingly, the present invention provides:

a method for predicting sensitivity of a cancer cell to a GPX4 inhibitor, the method comprising:
(a) detecting the presence or absence of a suppressed function of a SWI/SNF complex factor in a cancer cell; and
(b) predicting a cancer cell having the suppressed function of a SWI/SNF complex factor detected, as having sensitivity to a GPX4 inhibitor.
(Hereinafter, the method is sometimes referred to as a "cancer cell sensitivity prediction method".)

According to the above-described finding, sensitivity to treatment with a GPX4 inhibitor can be predicted using a suppressed function of a SWI/SNF complex factor as an indicator. Accordingly, the present invention provides:
a method for predicting sensitivity of a cancer patient to treatment with a GPX4 inhibitor, the method comprising:
(a) detecting the presence or absence of a suppressed function of a SWI/SNF complex factor in a cancer cell contained in a cancer patient-derived sample; and
(b) predicting a cancer patient having the suppressed function of a SWI/SNF complex factor detected in the cancer cell, as having sensitivity to treatment with a GPX4 inhibitor.
(Hereinafter, the method is sometimes referred to as a "cancer patient sensitivity prediction method".)

Further, a patient having a suppressed function of a SWI/SNF complex factor detected in this way can be suitable for cancer treatment with a GPX4 inhibitor, and therefore using the suppressed function of a SWI/SNF complex factor as an indicator, a patient who benefits from cancer treatment with a GPX4 inhibitor and a patient who does not benefit from the cancer treatment can be discriminated from each other to perform efficient treatment. Accordingly, the present invention provides:
a method for selecting a cancer patient for cancer treatment with GPX4 inhibitor, the method comprising:
(a) detecting the presence or absence of a suppressed function of a SWI/SNF complex factor in a cancer cell contained in a cancer patient-derived sample; and
(b) selecting a cancer patient having the suppressed function of a SWI/SNF complex factor detected in the cancer cell, for cancer treatment with a GPX4 inhibitor.
(Hereinafter, the method is sometimes referred to as a "cancer patient selection method".)

### (Samples)

In the present invention, malignant neoplasms such as carcinomas (epithelial tumors), leukemia, malignant lymphomas, myelomas, sarcomas and carcinosarcomas, are referred to collectively as "cancer" or "tumor", and a cell forming the cancer is referred to as a "cancer cell". The cancer containing a cancer cell in which the presence or absence of a suppressed function of a SWI/SNF complex factor can be detected is not particularly limited, and examples thereof include lung cancer, ovary cancer, uterus cancer, liver cancer, stomach cancer, esophagus cancer, bowel cancer, pancreas cancer, prostate cancer, bladder cancer and kidney cancer.

In the present invention, the "cancer patient" may be not only a human affected with the cancer, but also a human possibly affected with the cancer. In the method of the present invention, the cancer patient to be subjected to detection of a suppressed function of a SWI/SNF complex factor is not particularly limited, and may include all cancer patients.

The "cancer patient-derived sample" for use in the present invention is not particularly limited as long as it is a biological sample allowing the presence or absence of suppressed function of a SWI/SNF complex factor to be detected, and a specimen material such as a cancer biopsy specimen material, blood, urine, body cavity fluid or tumor cell-derived circulating DNA (ctDNA). The cancer patient-derived sample may be a protein extract or a nucleic acid extract obtained from the specimen material (e.g. a mRNA extract, or a cDNA preparation or a cRNA preparation prepared from a mRNA extract). In the present description, the "biological sample" includes cancer patient-derived samples and cancer cell culture-derived samples.

### (GPX4 inhibitor)

The "GPX4 inhibitor" in the present invention means a composition containing at least one compound which inhibits GPX4 (glutathione peroxidase 4). The GPX4 inhibitor may be one including only the compounds or a combination thereof, or one further containing additive ingredients described below. The "compound which inhibits GPX4" in the present invention includes compounds which inhibit at least one of the activity of GPX4 and the expression of GPX4.

The "GPX4 (herein, sometimes referred to as "GPX4 protein") targeted by the GPX4 inhibitor in the present invention is one of isozymes of glutathione peroxidase having peroxidase activity in which the peroxide structure of glutathione is oxidized and cleaved to be broken down into two hydroxy groups (eight types: GPX 1 to GPX 8 are currently known). GPXs other than GPX4 can reduce hydrogen peroxide and peroxidized fatty acids as substrates, whereas GPX4 is an enzyme which also has a function of directly reducing peroxidized phospholipid. A nucleotide sequence of human-derived typical DNA (cDNA) encoding GPX4 is set forth as SEQ ID NO: 1 (NCBI reference number: NM_002085.5), and a typical amino acid sequence of human-derived GPX4 protein is set forth as SEQ ID NO: 2 (NCBI reference number: NP_002076.2). Even among DNAs encoding GPX4 which does not have a mutation associated with substitution, deletion, insertion, addition and the like of amino acid sequences, there may be an interindividual difference in sequence due to polymorphism or the like.

Inhibition of the activity of GPX4 by the compound can be confirmed by, for example, adding phosphatidylcholine hydroperoxide, which is a substrate of GPX4, to a lysate of a cell treated with a test compound, and using reduction thereof as an indicator (e.g. Viswanathan et al., Nature.2017 July 27; 547 (7664): 453-457, Dependency of a therapy-resistant state of cancer cells on a lipid peroxidase pathway, and Yang et al., Cell. 2014 January 16 156 (0): 317-331, Regulation of Ferroptotic Cancer Cell Death by GPX4). When the activity of GPX4 is inhibited by the test compound, reduction of the substrate does not occur, or hardly occurs (the amount of reduction decreases).

Inhibition of the activity of GPX4 by the compound can also be confirmed by, for example, treating a purified GPX4 preparation with a test compound, adding GSH and a peroxide which are substrates of GPX4, glutathione reductase and NADPH, and measuring activity by an enzyme recycling method (e.g. Shinome et al., Antioxid. Redox Signal.22 (4), 281-293, Expression of Inactive Glutathione Peroxidase 4 Leads to Embryonic Lethality, and Inactivation of the Alox 15 Gene Does Not Rescue Such Knock-In Mice). When the activity of GPX4 is inhibited by the test compound, the amount of GSH or NADPH consumed decreases.

Inhibition of the expression of GPX4 by the compound can be confirmed by, for example, detecting a decrease in expression of GPX4 in a cell treated with the test compound. Examples of the method for detecting a decrease in expression of GPX4 include a method in which normally, the expression level of GPX4 is detected at a transcriptional level or a translational level, and compared to a control (e.g. expression level of a cell which is not treated with the test compound) to confirm that the expression level is lower than the control.

In the method for detecting the expression level of GPX4 at a transcriptional level, first, RNA or cDNA is prepared from a cell treated with the test compound. The method for extracting RNA from the cell is not particularly limited, and a known method can be appropriately selected and used. Examples thereof include extraction methods using phenol and a chaotropic salt (more specifically, extraction methods using a commercially available kit such as TRIzol (manufactured by Invitrogen Corporation) or ISOGEN (manufactured by Wako Pure Chemical Industries, Ltd.)), and methods using another commercially available kit (e.g. RNAPrep Total RNA Extraction Kit (manufactured by Beckman Coulter Inc.), RNeasy Mini (manufactured by QIAGEN N.V.) or RNA Extraction Kit (manufactured by Pharmacia Biotech, Inc.)). Further, the reverse transcriptase used for preparation of cDNA from extracted RNA is not particularly limited, and examples thereof include reverse transcriptases derived from retroviruses such as RAV (Rous associated virus) and AMV (Avian myeloblastosis virus), and reverse transcriptases derived from mouse retroviruses such as MMLV (Moloney murine leukemia virus).

Subsequently, an oligonucleotide primer or an oligonucleotide probe is used for an amplification reaction or a hybridization reaction, and an amplified product or a hybrid product thereof is detected. As such a method, for example, a RT-PCR method, a Northern blot method, a dot blot method, a DNA array method, an in situ hybridization method, a RNase protection assay method, mRNA-seq or the like can be used. Those skilled in the art can design an oligonucleotide primer or an oligonucleotide probe suitable for each method in a conventional manner on the basis of a nucleotide sequence of cDNA encoding GPX4.

In the method for detecting the expression level of GPX4 at a translational level, first, a protein sample is prepared from a cell treated with the test compound. Subsequently, using an antibody specific to GPX4 protein, an antigen-antibody reaction is carried out, and GPX4 protein is detected. In such a method for detecting protein using an antibody, for example, an antibody specific to GPX4 protein is added to the protein sample to carry out an antigen-antibody reaction, and binding of the antibody to GPX4 protein is detected. When the sample is labeled with antibody specific to GPX4 protein, GPX4 protein can be directly detected, and when the sample is not labeled, a labeled molecule which recognizes the antibody (e.g. secondary antibody or protein A) can be further applied to indirectly detect GPX4 protein using the label of the molecule. As such a method, for example, an immunohistochemistry (immunostaining) method, a Western blotting method, an ELISA method, flow cytometry, imaging cytometry, radioimmunoassay, an immunoprecipitation method, or an analysis method using an antibody array can be used.

The type, the origin and the like of an antibody used are not particularly limited, and a monoclonal antibody is preferable. An oligoclonal antibody (mixture of several antibodies or dozens of antibodies) or a polyclonal antibody can also be used as long as it is possible to detect GPX4 protein with sufficient specificity. Functional fractions of antibodies such as Fab, Fab', F(ab')₂, Fv, scFv, sc(Fv)₂, dsFv and diabodies, and multimers (e.g. dimers, trimers, tetramers and polymers) thereof can also be used. Such an anti-GPX4 protein antibody may be a marketed product.

The GPX4 protein can also be detected by mass spectrometry (MS). In particular, analysis by a mass spectrometer coupled with liquid chromatography (LC/MS) is sensitive, and therefore advantageous. Detection by mass spectrometry can be performed by, for example, labeling the protein sample with the protein, fractionating the labeled protein, subjecting the fractionated protein to mass analysis, and identifying GPX4 protein from the mass analysis value. As the label, an isotopic labeling reagent known in the art can be used, and an appropriate labeling reagent can be obtained as a marketed product. The fractionation can be performed by a method known in the art, and for example, a commercially available ionexchange column or the like can be used.

The "compound which inhibits GPX4" in the present invention is not particularly limited, may be a known compound, or a compound identified by screening described later, and is preferably at least one selected from the group consisting of an organic or inorganic compound molecule, a polypeptide and a polynucleotide.

Examples of the compound molecule include low-molecular compounds (molecular weight: less than 800) and middle-molecular compounds (molecular weight: 800 to 2,000). The polypeptide includes full-length polypeptides encoded by a gene, fractions thereof, synthesized polypeptides, cyclic polypeptides and glycopeptides. The polypeptide includes antibodies and antigen peptides, and the antibody may be a polyclonal antibody or a monoclonal antibody. The antibody includes complete antibodies, antibody fractions (e.g. Fab, Fab', F(ab')₂, Fv, scFv, sc(Fv)₂, dsFv and diabodies), multimers thereof, and low-molecular antibodies in which variable regions of antibodies are bound. Examples of the polynucleotide include DNA, RNA and SiRNA, including full-length polynucleotides, fractions thereof and synthesized polynucleotides.

The "GPX4 inhibitor" in the present invention can be used as various dosage forms such as tablets, pills, powders, granules, capsules and solutions depending on the properties thereof, may further contain pharmacologically acceptable additive ingredients such as sterilized water, physiological saline, vegetable oil, solvents, bases, emulsifiers, suspension agents, surfactants, stabilizers, flavoring agents, fragrances, excipients, vehicles, preservatives, binders, diluents, tonicity agents, soothing agents, extenders, disintegrants, buffering agents, coating agents, lubricants, colorants, sweeteners, viscous agents, taste and odor improvers and solubilizers depending on the dosage form, and can be produced by a known pharmaceutical method using these components.

In the GPX4 inhibitor according to the present invention, the content of the compound which inhibits the GPX4 (the total content of the compounds if there are the two or more compounds) can be appropriately adjusted according to the dosage form or the use purpose of the GPX4 inhibitor.

### (Suppressed function of SWI/SNF complex factor)

### [SWI/SNF complex factor]

The "SWI/SNF complex" in the present invention, which is one of chromatin modeling factors, is a complex having mainly a function of converting a chromatin structure by moving and removing nucleosome with the utilization of energy from ATP hydrolysis, and includes a plurality of subunits.

The "SWI/SNF complex factor (herein, sometimes referred to as "SWI/SNF complex factor protein")" in the present invention is a subunit forming the SWI/SNF complex, and examples thereof include SMARCA2 (herein, sometimes referred to as "SMARCA2 protein"; hereinafter, the same applies to subsequent factors), SMARCA4, ARID1A, ARID1B, ARID2, ACTL6A, ACTL6B, DPF1, DPF2, DPF3, SMARCB1, SMARCC1, SMARCC2, SMARCD1, SMARCD2, SMARCD3, SMARCE1, SS18, SS18L1, BCL7A, BCL7B, BCL7C, BCL11A, BCL11B, BRD7, BRD9, PBRM1 and PHF10. The SWI/SNF complex factor in which a suppressed function is detected may be one of the above-mentioned factors alone, or a combination of two or more of the above-mentioned factors.

Among the SWI/SNF complex factors, SMARCA2, SMARCA4, SMARCC1, SMARCC2, SMARCB1, ARID1A, ARID1B, SMARCD1, SMARCD2, SMARCD3, BCL11B, SMARCE1, PHF10, DPF1, DPF3, ACTL6A and ACTL6B are BAF complex factors forming the BAF complex, and SMARCA4, SMARCC1, SMARCC2, SMARCB1, ARID2, PBRM1, BRD7, BCL11B, SMARCE1, PHF10, DPF1, DPF3, ACTL6A and ACTL6B are PBAF complex factors forming the PBAF complex. The SWI/SNF complex factor according to the present invention is preferably at least one selected from the group consisting of SMARCA2, SMARCA4, ARID1A, ARID1B, ARID2 and BCL11B, more preferably at least one selected from the group consisting of BAF complex factors, still more preferably at least one selected from the group consisting of SMARCA2, SMARCA4, ARID1A, ARID1B and BCL11B, among the above-mentioned factors.

For the above-described SWI/SNF complex factors, Tables 1 to 3 show the sequence numbers of nucleotide sequences of human-derived typical DNAs (cDNAs) encoding the SWI/SNF complex factors, and the sequence numbers of typical amino acid sequences of the human-derived SWI/SNF complex factor proteins, but the sequence numbers are not limited thereto. Tables 1 to 3 also show names and reference IDs in NCBI RefSeq Database (National Center for Biotechnology Information Reference Sequence Database) for the SWI/SNF complex factors. Even among DNAs encoding a SWI/SNF complex factor which does not have a mutation associated with substitution, deletion, insertion, addition and the like of amino acid sequences, there may be an interindividual difference in sequence due to polymorphism or the like.

**[Table 1]**

| SEQ ID NO: | Nucleotide sequence/amino acid sequence | Name (SWI/SNF complex factor) | Name (NCBI) | NCBI Reference ID |
|---|---|---|---|---|
| 3 | Nucleotide sequence (cDNA) | SMARCA2 | Homo sapiens SWI/SNF related, matrix associated, actin dependent regulator of chromatin, subfamily a, member 2 (SMARCA2), transcript variant 3, mRNA | NM_001289396.1 |
| 4 | Amino acid sequence | SMARCA2 | probable global transcription activator SNF2L2 isoform a | NP_001276325.1 |
| 5 | Nucleotide sequence (cDNA) | SMARCD2 | Homo sapiens SWI/SNF related, matrix associated, actin dependent regulator of chromatin, subfamily d, member 2 (SMARCD2), transcript variant 1, mRNA | NM_001098426.2 |
| 6 | Amino acid sequence | SMARCD2 | SWI/SNF-related matrix-associated actin-dependent regulator of chromatin subfamily D member 2 isoform 1 | NP_001091896.1 |
| 7 | Nucleotide sequence (cDNA) | SMARCA4 | Homo sapiens SWI/SNF related, matrix associated, actin dependent regulator of chromatin, subfamily a, member 4 (SMARCA4), transcript variant 1, mRNA | NM_001128849.3 |
| 8 | Amino acid sequence | SMARCA4 | transcription activator BRG1 isoform A [Homo sapiens] | NP_001122321.1 |
| 9 | Nucleotide sequence (cDNA) | SMARCD3 | Homo sapiens SWI/SNF related, matrix associated, actin dependent regulator of chromatin, subfamily d, member 3 (SMARCD3), transcript variant 3, mRNA | NM_001003801.2 |
| 10 | Amino acid sequence | SMARCD3 | SWI/SNF-related matrix-associated actin-dependent regulator of chromatin subfamily D member 3 isoform 2 | NP_001003801.1 |
| 11 | Nucleotide sequence (cDNA) | ARID1A | Homo sapiens AT-rich interaction domain 1A (ARID1A), transcript variant 1, mRNA | NM_006015.6 |
| 12 | Amino acid sequence | ARID1A | AT-rich interactive domain-containing protein 1A isoform a | NP_006006.3 |
| 13 | Nucleotide sequence (cDNA) | SMARCE1 | Homo sapiens SWI/SNF related, matrix associated, actin dependent regulator of chromatin, subfamily e, member 1 (SMARCE1), mRNA | NM_003079.5 |
| 14 | Amino acid sequence | SMARCE1 | SWI/SNF-related matrix-associated actin-dependent regulator of chromatin subfamily E member 1 | NP_003070.3 |
| 15 | Nucleotide sequence (cDNA) | ARID1B | Homo sapiens AT-rich interaction domain 1 B (ARID1 B), transcript variant 2, mRNA | NM_001374820.1 |
| 16 | Amino acid sequence | ARID1B | AT-rich interactive domain-containing protein 1B isoform 2 | NP_001361749.1 |
| 17 | Nucleotide sequence (cDNA) | SS18 | Homo sapiens SS18 subunit of BAF chromatin remodeling complex (SS18), transcript variant 1, mRNA | NM_001007559.3 |
| 18 | Amino acid sequence | SS18 | protein SSXT isoform 1 | NP_001007560.1 |
| 19 | Nucleotide sequence (cDNA) | ARID2 | Homo sapiens AT-rich interaction domain 2 (ARID2), transcript variant 1, mRNA | NM_152641.4 |
| 20 | Amino acid sequence | ARID2 | AT-rich interactive domain-containing protein 2 isoform 1 | NP_689854.2 |

**[Table 2]**

| SEQ ID NO: | Nucleotide sequence/amino acid sequence | Name (SWI/SNF complex factor) | Name (NCBI) | NCBI Reference ID |
|---|---|---|---|---|
| 21 | Nucleotide sequence (cDNA) | SS18L1 | Homo sapiens SS18L1 subunit of BAF chromatin remodeling complex (SS18L1), transcript variant 1, mRNA | NM_198935.3 |
| 22 | Amino acid sequence | SS18L1 | calcium-responsive transactivator isoform 1 | NP_945173.1 |
| 23 | Nucleotide sequence (cDNA) | ACTL6A | Homo sapiens actin like 6A (ACTL6A), transcript variant 1, mRNA | NM_004301.5 |
| 24 | Amino acid sequence | ACTL6A | actin-like protein 6A isoform 1 | NP_004292.1 |
| 25 | Nucleotide sequence (cDNA) | BCL7A | Homo sapiens BAF chromatin remodeling complex subunit BCL7A (BCL7A), transcript variant 1, mRNA | NM_020993.5 |
| 26 | Amino acid sequence | BCL7A | B-cell CLL/lymphoma 7 protein family member A isoform a | NP_066273.1 |
| 27 | Nucleotide sequence (cDNA) | ACTL6B | Homo sapiens actin like 6B (ACTL6B), transcript variant 1, mRNA | NM_016188.5 |
| 28 | Amino acid sequence | ACTL6B | actin-like protein 6B | NP_057272.1 |
| 29 | Nucleotide sequence (cDNA) | BCL7B | Homo sapiens BAF chromatin remodeling complex subunit BCL7B (BCL7B), transcript variant 1, mRNA | NM_001707.4 |
| 30 | Amino acid sequence | BCL7B | B-cell CLL/lymphoma 7 protein family member B isoform 1 | NP_001698.2 |
| 31 | Nucleotide sequence (cDNA) | DPF1 | Homo sapiens double PHD fingers 1 (DPF1), transcript variant 1, mRNA | NM_001135155.2 |
| 32 | Amino acid sequence | DPF1 | zinc finger protein neuro-d4 isoform a | NP_001128627.1 |
| 33 | Nucleotide sequence (cDNA) | BCL7C | Homo sapiens BAF chromatin remodeling complex subunit BCL7C (BCL7C), transcript variant 2, mRNA | NM_004765.4 |
| 34 | Amino acid sequence | BCL7C | B-cell CLL/lymphoma 7 protein family member C isoform 2 | NP_004756.2 |
| 35 | Nucleotide sequence (cDNA) | DPF2 | Homo sapiens double PHD fingers 2 (DPF2), transcript variant 1, mRNA | NM_006268.5 |
| 36 | Amino acid sequence | DPF2 | zinc finger protein ubi-d4 isoform 1 | NP_006259.1 |
| 37 | Nucleotide sequence (cDNA) | BCL11A | Homo sapiens BAF chromatin remodeling complex subunit BCL11A (BCL1 1A), transcript variant 1, mRNA | NM_022893.4 |
| 38 | Amino acid sequence | BCL11A | B-cell lymphoma/leukemia 11A isoform 1 | NP_075044.2 |
| 39 | Nucleotide sequence (cDNA) | DPF3 | Homo sapiens double PHD fingers 3 (DPF3), transcript variant 2, mRNA | NM_001280542.1 |
| 40 | Amino acid sequence | DPF3 | zinc finger protein DPF3 isoform 2 | NP_001267471.1 |

**[Table 3]**

| SEQ ID NO: | Nucleotide sequence/amino acid sequence | Name (SWI/SNF complex factor) | Name (NCBI) | NCBI Reference ID |
|---|---|---|---|---|
| 41 | Nucleotide sequence (cDNA) | BCL11B | Homo sapiens BAF chromatin remodeling complex subunit BCL11B (BCL11B), transcript variant 1, mRNA | NM_138576.4 |
| 42 | Amino acid sequence | BCL11B | B-cell lymphoma/leukemia 11B isoform 1 | NP_612808.1 |
| 43 | Nucleotide sequence (cDNA) | SMARCB1 | Homo sapiens SWI/SNF related, matrix associated, actin dependent regulator of chromatin, subfamily b, member 1 (SMARCB1), transcript variant 1, mRNA | NM_003073.5 |
| 44 | Amino acid sequence | SMARCB1 | SWI/SNF-related matrix-associated actin-dependent regulator of chromatin subfamily B member 1 isoform a | NP_003064.2 |
| 45 | Nucleotide sequence (cDNA) | BRD7 | Homo sapiens bromodomain containing 7 (BRD7), transcript variant 1, mRNA | NM_001173984.3 |
| 46 | Amino acid sequence | BRD7 | bromodomain-containing protein 7 isoform 1 | NP_001167455.1 |
| 47 | Nucleotide sequence (cDNA) | SMARCC1 | Homo sapiens SWI/SNF related, matrix associated, actin dependent regulator of chromatin subfamily c member 1 (SMARCC1), mRNA | NM_003074.4 |
| 48 | Amino acid sequence | SMARCC1 | SWI/SNF complex subunit SMARCC1 | NP_003065.3 |
| 49 | Nucleotide sequence (cDNA) | BRD9 | Homo sapiens bromodomain containing 9 (BRD9), transcript variant 1, mRNA | NM_023924.5 |
| 50 | Amino acid sequence | BRD9 | bromodomain-containing protein 9 isoform 1 | NP_076413.3 |
| 51 | Nucleotide sequence (cDNA) | SMARCC2 | Homo sapiens SWI/SNF related, matrix associated, actin dependent regulator of chromatin subfamily c member 2 (SMARCC2), transcript variant 3, mRNA | NM_001130420.3 |
| 52 | Amino acid sequence | SMARCC2 | SWI/SNF complex subunit SMARCC2 isoform c | NP_001123892.1 |
| 53 | Nucleotide sequence (cDNA) | PBRM1 | Homo sapiens polybromo 1 (PBRM1), transcript variant 2, mRNA | NM_018313.5 |
| 54 | Amino acid sequence | PBRM1 | protein polybromo-1 isoform 2 | NP_060783.3 |
| 55 | Nucleotide sequence (cDNA) | SMARCD1 | Homo sapiens SWI/SNF related, matrix associated, actin dependent regulator of chromatin, subfamily d, member 1 (SMARCD1), transcript variant 1, mRNA | NM_003076.5 |
| 56 | Amino acid sequence | SMARCD1 | SWI/SNF-related matrix-associated actin-dependent regulator of chromatin subfamily D member 1 isoform a | NP_003067.3 |
| 57 | Nucleotide sequence (cDNA) | PHF10 | Homo sapiens PHD finger protein 10 (PHF10), transcript variant 1, mRNA | NM_018288.4 |
| 58 | Amino acid sequence | PHF10 | PHD finger protein 10 isoform a | NP_060758.2 |

The "suppressed function of a SWI/SNF complex factor" in the present invention includes a decrease in activity, including inactivation of a SWI/SNF complex containing the factor (having the factor as a constituent factor), and a decrease in expression of a SWI/SNF complex factor.

### [Detection of decrease in activity of SWI/SNF complex]

The "decrease in activity of a SWI/SNF complex" means that the intrinsic activity of the SWI/SNF complex decreases, and normally means that the activity level is lower than a control (e.g. activity level in a healthy subject or a non-cancer tissue of the same patient). The "decrease in activity of a SWI/SNF complex" includes both inactivation and a decrease in activity of all or part of the SWI/SNF complex.

### [Direct detection of decrease in activity of SWI/SNF complex]

The method for "direct detection of a decrease in activity of a SWI/SNF complex" in the present invention is not particularly limited, and a decrease in activity of a SWI/SNF complex can be confirmed by, for example, purifying a SWI/SNF complex from a biological sample by a method such as immunoprecipitation and measuring chromatin remodeling activity (e.g. Michael L. Phelan et al., Molecular Cell, Vol.3, p. 247-253, February, 1999, Reconstitution of a Core Chromatin Remodeling Complex from SWI/SNF Subunits). When the activity is lower than a control, it can be determined that a decrease in activity of the SWI/SNF complex is detected.

As a method for directly detecting a decrease in activity of the SWI/SNF complex, for example, the decrease in activity can also be confirmed by measuring ATPase activity. For example, a SWI/SNF complex is purified from a biological sample by a method such as immunoprecipitation, and using a marketed product such as ADP-glo (manufactured by Promega Corporation), the amount of ATP consumed by the complex is detected. When the result of the detection shows that the amount of ATP consumed is lower than a control, it can be determined a decrease in activity of the SWI/SNF complex is detected.

### <Detection of mutation of gene for SWI/SNF complex factor>

The decrease in activity of a SWI/SNF complex is typically caused by a loss-of-function mutation in a gene for a SWI/SNF complex factor forming the complex (DNA encoding a SWI/SNF complex factor). Thus, a mutation of a gene for a SWI/SNF complex factor can be detected as a decrease in activity of the SWI/SNF complex.

The loss-of-function mutation can be caused by, for example, a missense mutation in a gene for a SWI/SNF complex factor, a nonsense mutation over the entire region, a frameshift mutation, total or partial deletion of a gene, or the like, but is not limited thereto as long as the activity of the SWI/SNF complex is decreased. Table 4 below shows examples of a mutation of a gene for a SWI/SNF complex factor in main cancer cells. Table 4 also shows examples of the presence or absence of a SWI/SNF complex factor in main cancer cells.

| Cell line | Tissue | Status | Status of SWVSNF component | | | |
|---|---|---|---|---|---|---|
| | | | mutation | Reference | protein expression | Reference |
| NC I-H358 | Lung | Adenocarcinoma | - | - | - | - |
| NCI-H2122 | Lung | Adenoca rci noma | - | - | - | - |
| NCI-H2110 | Lung | Carcinoma, Non-Small-Cell Lung | - | - | - | - |
| NCI-H1792 | Lung | Adenoca rci noma | - | - | - | - |
| NCI-H1437 | Lung | Adenoca rci noma | - | - | - | - |
| MOR | Lung | Adenoca rci noma | - | - | - | - |
| COV362 | Ovary | Carcinoma | - | - | - | - |
| MKN-45 | Stomach | Adenoca rci noma | - | - | - | - |
| NCI-H838 | Lung | Adenoca rci noma | BCL11B : p.E508* | CCLE | SMARCA2 protein: + | Hoffman et al. 2014 |
| | | | | | SMARCA4 protein: - | Hoffman et al. 2014 |
| B1203L | Lung | Adenoca rci noma | ARID1A : p.Q1417* | In this patent | - | - |
| | | | ARID2 : p.R572* | In this patent | | |
| SBC-5 | Lung | Carcinoma, Small Cell | SMARCA4 : p.RR1243fs | CCLE | SMARCA2 protein: - | Hoffman et al. 2014 |
| | | | | | SMARCA4 protein: - | Hoffman et al. 2014 |
| TOV-21G | Ovary | Adenocarcinoma, Clear Cell | ARID1A : p.N756fs, p.P549fs | CCLE | ARID1A protein: - | Ogiwara etal. 2019 |
| NCI-H1703 | Lung | Carcinoma, Squamous Cell | SMARCA4 : Splie site | CCLE | SMARCA2 protein: - | Matsubara et al. 2012 |
| | | | | | SMARCA4 protein: - | Matsubara et al. 2012 |
| SNU-1327 | Lung | Adenoca rci noma | SMARCA4 : p.Q160* | In this patent | - | - |
| NCI-H23 | Lung | Adenoca rci noma | SMARCA4 : p.K1566N, p.E1567* | CCLE | SMARCA2 protein: - | Matsubara et al. 2012 |
| | | | | | SMARCA4 protein : - | Matsubara et al. 2012 |
| OVISE | Ovary | Adenocarcinoma, Clear Cell | ARID1A : p.H203fs, p.Q543fs | CCLE | ARID1A protein: - | Ogiwara et al. 2019 |
| | | | ARID1B : p.Y827* | CCLE | | |
| NC I-H522 | Lung | Adenoca rci noma | SMARCA4 : p.P270fs | CCLE | SMARCA2 protein: - | Matsubara et al. 2012 |
| | | | | | SMARCA4 protein: - | Matsubara et al. 2012 |
| SK-HEP-1 | Liver | Adenoca rci noma | SMARCA4 : p.E1582* | CCLE | SMARCA2 protein: + | Hoffman et al. 2014 |
| | | | | | SMARCA4 protein: - | Hoffman et al. 2014 |

In Table 4, "Reference" indicates databases, documents and the like in which relevant gene mutations (mutation) or presence or absence of expression (protein expression) are reported, "CCLE" indicates a database prepared by Broad Institute: "Cancer Cell Line Encyclopedia (https://portals.broadinstitute.org/ccle), "Hoffman et al. 2014" indicates the document: "Hoffman et al., PNAS February 25, 2014 111 (8), p. 3128-3133", "Matsubara et al., 2012" indicates the document: "Matsubara et al., Cancer Sci, February 2013, vol. 104, No. 2, p. 266-273", "Ogiwara et al., 2019" indicates the document: Ogiwara et al., Volume 35, Issue 2, 11 February 2019, p. 177-190. e8", and "In this patent" indicates that the present inventors have confirmed the presence of a truncating mutation as information of mutation or expression which has not been reported in the literature.

In addition, specific examples of the mutation of a gene for a SWI/SNF complex factor which causes a decrease in activity of a SWI/SNF complex include, but are not limited to, ARID1A: p. Y551Lfs ^{∗} 72 (Insertion-Frameshift) (COSMIC Legacy Mutation ID: COSM 51423).

The method for "detection of a mutation of a gene for a SWI/SNF complex factor" in the present invention is not particularly limited, and examples thereof include the following method.

In the present invention, the phrase "detecting a mutation" means that a mutation on genome DNA is detected in principle. When the mutation on genome DNA is reflected in a change of a base in a transcription product or a change of an amino acid in a translation product, the meaning also includes detection of the change of the transcription product or the translation product (i.e. indirect detection).

The preferred aspect of the method of the present invention is a method for detecting a mutation by directly determining a nucleotide sequence of the region of a gene for a SWI/SNF complex factor in a cancer cell. In the present invention, the "region of a gene for a SWI/SNF complex factor" means a certain region on genome DNA which contains a gene for a SWI/SNF complex factor. The regions also each independently include, in addition to translated regions, untranslated regions such as an expression control region for the relevant gene (e.g. promotor region or enhancer region) and a 3'-end untranslated region for the relevant gene.

In this method, first, a DNA sample is prepared from a biological sample. Examples of the DNA sample include genome DNA samples, and cDNA samples prepared by reverse transcription from RNA.

The method for extracting genome DNA or RNA from a biological sample is not particularly limited, and a known method can be appropriately selected and used. Examples of the method for extracting genome DNA include a SDS phenol method (a method in which protein of a tissue stored in a urea-containing solution or ethanol is denatured with a proteinase (proteinase K), a surfactant (SDS) and phenol, and DNA is precipitated and extracted from the tissue with ethanol), and DNA extraction methods using Clean Columns (registered trademark, manufactured by NexTec Co., Ltd.), AquaPure (registered trademark, manufactured by Bio-Rad Laboratories, Inc.), ZR Plant/Seed DNA Kit (manufactured by Zymo Research), AquaGenomicSolution (registered trademark, manufactured by Mo Bi Tec GmbH), prepGEM (registered trademark, manufactured by ZyGEM LLC) and BuccalQuick (registered trademark, manufactured by TrimGen Corporation).

Examples of the method for extracting RNA from a biological sample and the method for preparing cDNA from the extracted RNA include methods similar to the above-mentioned method for detecting the expression level of GPX4 at a transcriptional level.

In this aspect, subsequently, DNA containing the region of a gene for a SWI/SNF complex factor is isolated, and the nucleotide sequence of the isolated DNA is determined. The isolation of DNA can be performed by PCR with genome DNA or RNA as a template, or the like using a pair of oligonucleotide primers designed to sandwich all or part of the region of a gene for a SWI/SNF complex factor. The determination of the nucleotide sequence of the isolated DNA can be performed by a method known to those skilled in the art, such as a Maxam-Gilbert method or a Sanger method. For the isolated DNA and the extracted DNA, it is also possible to use a next-generation sequencer which enables analysis such that nucleotide sequences of genes can be read rapidly and exhaustively, etc.

By comparing the determined nucleotide sequence of DNA or cDNA (for example, when the biological sample is a cancer patient-derived sample, the nucleotide sequence of DNA or cDNA derived from a non-cancer tissue of the same patient, or a known database), the presence or absence of a mutation in the region of a gene for a SWI/SNF complex factor in a cancer cell of the biological sample can be determined.

As the method for detecting a mutation in the region of a gene for a SWI/SNF complex factor, various methods capable of detecting a mutation can be used in addition to methods for directly determining the nucleotide sequence of DNA or cDNA.

For example, the detection of a mutation in the present invention can also be performed by the following method. First, a DNA or cDNA sample is prepared from a biological sample. Subsequently, an oligonucleotide probe is prepared which has a nucleotide sequence complementary to a nucleotide sequence containing a mutation site of the region of a gene for a SWI/SNF complex factor and is labeled with a reporter fluorescent dye and a quencher fluorescent dye. The oligonucleotide probe is hybridized to the DNA or cDNA sample, and the nucleotide sequence containing the mutation site of the region of a gene for a SWI/SNF complex factor is amplified using as a template the DNA or cDNA sample to which the oligonucleotide probe is hybridized. Fluorescence generated by the reporter fluorescent dye due to degradation of the oligonucleotide probe which is caused by the amplification is detected, and the detected fluorescence is then compared to a control. Examples of such a method include a double-dye probe method, so called a TaqMan (registered trademark) probe method.

In still another method, a DNA or cDNA sample is prepared from a biological sample. Subsequently, in a reaction system containing an intercalator which generated fluorescence when inserted between DNA double strands, a nucleotide sequence containing a mutation site of the region of a gene for a SWI/SNF complex factor is amplified using the DNA or cDNA sample as a template. The temperature of the reaction system is changed, a variation in intensity of fluorescence generated by the intercalator is detected, and the variation in intensity of the fluorescence with the detected change in temperature is compared to a control. Examples of such a method include a HRM (high resolution melting) analysis method.

In still another method, first, a DNA or cDNA sample is prepared from a biological sample. Subsequently, DNA containing all or part of the region of a gene for a SWI/SNF complex factor is amplified. Further, the amplified DNA is cleaved by a restriction enzyme. Subsequently, DNA fragments are separated according to the sizes thereof. Subsequently, the size of the detected DNA fragment is compared to a control. Examples of such a method include methods utilizing restriction fragment length polymorphism (RFLP), and a PCR-RFLP method.

In still another method, first, a DNA or cDNA sample is prepared from a biological sample. Subsequently, DNA containing all or part of the region of a gene for a SWI/SNF complex factor is amplified. Further, the amplified DNA is dissociated into single-stranded DNA. Subsequently, the dissociated single-stranded DNA is separated on a non-denaturing gel. The mobility of the separated single-stranded DNA on the gel is compared to a control. Examples of such a method include a PCR-SSCP (single-strand conformation polymorphism) method.

In still another method, first, a DNA or cDNA sample is prepared from a biological sample. Subsequently, DNA containing all or part of the region of a gene for a SWI/SNF complex factor is amplified. Further, the amplified DNA is separated on a gel in which the concentration of a DNA denaturant increases in steps. Subsequently, the mobility of the separated DNA on the gel is compared to a control. Examples of such a method include a denaturant gradient gel electrophoresis (DGGE) method.

As still another method, there is a method using DNA prepared from a biological sample and containing a mutation site of the region of a gene for a SWI/SNF complex factor, and a substrate on which an oligonucleotide probe hybridized to the DNA is fixed. Examples of such a method include a DNA array method.

In still another method, first, a DNA or cDNA sample is prepared from a biological sample. An "oligonucleotide primer having a nucleotide sequence complementary to bases on the 3' side of the bases of all or part of the region of a gene for a SWI/SNF complex factor by one base and a nucleotide sequence on the 3' side thereof" is prepared. Subsequently, with the DNA as a template, a dNTP primer elongation reaction is carried out using the primer. Subsequently, the primer elongation reaction product is applied to a mass analyzer to perform mass measurement. Subsequently, the gene type is determined from the result of the mass measurement. Subsequently, the determined gene type is compared to a control. Examples of such a method include a MALDI-TOF/MS method.

In still another method, first, a DNA or cDNA sample is prepared from a biological sample. Subsequently, an oligonucleotide probe consisting of 5'-"nucleotide sequence complementary to the bases of all or part of the region of a gene for a SWI/SNF complex factor and a nucleotide sequence on the 5' side thereof"-"nucleotide sequence which is not hybridized to bases on the 3' side of all or part of the region of a gene for a SWI/SNF complex factor by one base and a nucleotide sequence on the 3' side thereof"-3' (flap) is prepared. An "oligonucleotide probe having a nucleotide sequence complementary to the bases of all or part of the region of a gene for a SWI/SNF complex factor and a nucleotide sequence on the 3' side thereof" is prepared. Subsequently, the two oligonucleotide probes are hybridized to the prepared DNA or cDNA sample. Subsequently, the hybridized DNA is cleaved by a single-stranded DNA cleavage enzyme to liberate the flap. The single-stranded DNA cleavage enzyme is not particularly limited, and examples thereof include cleavases. In this method, subsequently, an oligonucleotide probe which has a sequence complementary to the flap and is labeled with reporter fluorescence and quencher fluorescence is hybridized to the flap. Subsequently, the intensity of generated fluorescence is measured. Subsequently, the measured fluorescence intensity is compared to a control. Examples of such a method include an Invader method.

In still another method, first, a DNA or cDNA sample is prepared from a biological sample. Subsequently, DNA containing all or part of the region of a gene for a SWI/SNF complex factor is amplified. The amplified DNA is dissociated into single-stranded DNA, and only one strand is separated from the dissociated single-stranded DNA. Subsequently, an elongation reaction is carried out base by base from near the bases of all or part of the region of a gene for a SWI/SNF complex factor, pyrophosphoric acid generated at this time is enzymatically caused to emit light, and the intensity of emission is measured. The measured fluorescence intensity is compared to a control. Examples of such a method include a Pyrosequencing method.

In still another method, first, a DNA or cDNA sample is prepared from a biological sample. Subsequently, DNA containing all or part of the region of a gene for a SWI/SNF complex factor is amplified. Subsequently, an "oligonucleotide primer having a nucleotide sequence complementary to bases on the 3' side of the bases of all or part of the region of a gene for a SWI/SNF complex factor by one base and a nucleotide sequence on the 3' side thereof" is prepared. Subsequently, with the amplified DNA as a template, a single-base elongation reaction is carried out using the prepared primer in the presence of a fluorescently labeled nucleotide. The polarization degree of fluorescence is measured. Subsequently, the measured polarization degree of fluorescence is compared to a control. Examples of such a method include an AcycloPrime method.

In still another method, first, a DNA or cDNA sample is prepared from a biological sample. Subsequently, DNA containing all or part of the region of a gene for a SWI/SNF complex factor is amplified. Subsequently, an "oligonucleotide primer having a nucleotide sequence complementary to bases on the 3' side of the bases of all or part of the region of a gene for a SWI/SNF complex factor by one base and a nucleotide sequence on the 3' side thereof" is prepared. Subsequently, with the amplified DNA as a template, a single-base elongation reaction is carried out using the prepared primer in the presence of a fluorescently labeled nucleotide. Subsequently, the type of base used for the single-base elongation reaction is determined. Subsequently, the determined type of base is compared to a control. Examples of such a method include a SNuPE method.

When the mutation is associated with a change of an amino acid in SWI/SNF complex factor protein (e.g. substitution, deletion, insertion or addition), the sample prepared from the biological sample may be protein. Here, for detecting a mutation, a method using a molecule binding specifically to a site at which a change of amino acids occurs due to the mutation, peptide mass fingerprinting method (PMF), a protein sequencer (Edman degradation method), or the like can be used.

For example, in a method for detecting protein using an antibody, first, a protein sample is prepared from a biological sample. Subsequently, an antigen-antibody reaction is carried out using an antibody specific to SWI/SNF complex factor protein (anti-SWI/SNF complex factor protein antibody), and SWI/SNF complex factor protein is detected. As such a method for detecting protein using an antibody, a method similar to the above-mentioned method for detecting protein using an antibody in the method for detecting the expression level of GPX4 at a translational level can be adjusted to the SWI/SNF complex factor protein, and adopted as appropriate. This method also has an advantage that additional information such as a form or a distribution state of cancer cells in a tissue can also be obtained immunohistochemically.

The type, the origin and the like of an antibody used are not particularly limited, and a monoclonal antibody is preferable. An oligoclonal antibody (mixture of several antibodies or dozens of antibodies) or a polyclonal antibody can also be used as long as it is possible to detect SWI/SNF complex factor protein with sufficient specificity. Functional fractions of antibodies such as Fab, Fab', F(ab')₂, Fv, scFv, sc(Fv)₂, dsFv and diabodies, and multimers (e.g. dimers, trimers, tetramers and polymers) thereof can also be used. The anti-SWI/SNF complex factor protein antibody may be a marketed product.

The SWI/SNF complex factor protein can also be detected by mass spectrometry (MS). In particular, analysis by a mass spectrometer coupled with liquid chromatography (LC/MS) is sensitive, and therefore advantageous. As a method for detection by mass spectrometry, a method similar to the above-mentioned method for detection by mass spectrometry in the method for detecting the expression level of GPX4 at a translational level can be adjusted to the SWI/SNF complex factor protein, and adopted as appropriate.

### [Detection of decrease in expression of SWI/SNF complex factor]

The "decrease in expression of a SWI/SNF complex factor" normally means that the expression level is lower than a control (e.g. expression level in a healthy subject or a non-cancer tissue of the same patient). Examples of the method for detecting "a decrease in expression of a SWI/SNF complex factor" include a method in which the expression level of a SWI/SNF complex factor is detected at a transcriptional level or a translational level, and compared to the control.

In the method for detecting the expression level of a SWI/SNF complex factor at a transcriptional level, first, RNA or cDNA is prepared from a biological sample by the above-described method. Subsequently, an oligonucleotide primer or an oligonucleotide probe is used for an amplification reaction or a hybridization reaction, and an amplified product or a hybrid product thereof is detected. As such a method, a method similar to the above-mentioned method for detecting the expression level of GPX4 at a transcriptional level can be adjusted to the SWI/SNF complex factor, and adopted as appropriate.

In the method for detecting the expression level of a SWI/SNF complex factor at a translational level, first, a protein sample is prepared from a biological sample. Subsequently, an antigen-antibody reaction is carried out using an antibody specific to SWI/SNF complex factor protein, and SWI/SNF complex factor protein is detected. Such a method for detecting protein using an antibody is as described for the above-mentioned method for detecting SWI/SNF complex factor protein.

In the method for detecting the expression level of a SWI/SNF complex factor at a translational level, the SWI/SNF complex factor protein can also be detected by mass spectrometry (MS). Such a method for detection by mass spectrometry is as described for the above-mentioned method for detecting SWI/SNF complex factor protein.

It is known in the art that one of causes of a decrease in expression of a gene is excessive methylation of a promotor. Therefore, the presence or absence of a suppressed function of a SWI/SNF complex factor may be detected using methylation of a gene promotor for the SWI/SNF complex factor as an indicator. For detection of methylation of a promotor, it is possible to use, for example, a known method such as a method in which a change of a nucleotide sequence after treatment with bisulfite having an activity that converts methylated cytosine into uracil is directly detected by determination of the nucleotide sequence, or indirectly detected using a restriction endonuclease which can recognize (cleave) a nucleotide sequence before the bisulfite treatment and cannot recognize (cleave) a nucleotide sequence after the bisulfite treatment.

### (Prediction of sensitivity and selection of cancer patient)

Thus, if a suppressed function of a SWI/SNF complex factor is detected from a biological sample, and the biological sample is a cancer cell, the cancer cell can be predicted to have sensitivity to a GPX4 inhibitor, and if the biological sample is a cancer cell contained in a cancer patient-derived sample, a cancer patient having the mutation detected in the cancer cell can be predicted to have sensitivity to treatment with a GPX4 inhibitor, and the cancer patient can be selected for cancer treatment with a GPX4 inhibitor.

Here, the "sensitivity to a GPX4 inhibitor" and the "sensitivity to treatment with a GPX4 inhibitor" is an indicator of whether or not the GPX4 inhibitor can exhibit a therapeutic effect on a cancer cell. The sensitivity includes acceleration of death of cancer cells and suppression of growth of cancer cells by the GPX4 inhibitor. The prediction of sensitivity includes not only determination of the presence or absence of sensitivity, but also evaluation of whether sensitivity can be expected or not, etc., and evaluation of the degree of sensitivity when the sensitivity is present (e.g. evaluation of whether high sensitivity can be expected, moderate sensitivity can be expected, or the like). Therefore, a patient for cancer treatment may be selected in line with, for example, a level at which moderate sensitivity can be expected depending on the type and the degree of the suppressed function of a SWI/SNF complex factor.

On the other hand, if a suppressed function of a SWI/SNF complex factor is not observed in a cancer patient-derived sample, the patient can be excluded from subjects for cancer treatment with a GPX4 inhibitor. This enables improvement of the success ratio of the treatment.

### (GPX4)

Further, if GPX4 which is targeted by a GPX4 inhibitor according to the present invention is not normally expressed, it may be impossible to effectively perform cancer treatment with a GPX4 inhibitor. Therefore, in the cancer cell sensitivity prediction method, the cancer patient sensitivity prediction method and the cancer patient selection method, detection of a mutation and a decrease in expression of a gene for GPX4 can also be taken as an indicator.

As a method for detecting a mutation of a gene for GPX4, a method similar to the above-mentioned method for "detection of a gene for a SWI/SNF complex factor" can be adjusted to a mutation of a gene for GPX4, and adopted as appropriate. The method for detecting a decrease in expression of GPX4 is as described above.

### <Method for suppressing growth of cancer cells and method for treating cancer>

The present invention also provides:
a method for suppressing growth of cancer cells having a suppressed function of a SWI/SNF complex factor detected, the method comprising the step of:
contacting a GPX4 inhibitor with the cancer cell having the suppressed function of a SWI/SNF complex factor detected (herein, sometimes referred to as a "cancer cell growth inhibition method"); and a method for treating cancer, the method comprising the steps of:
(a) detecting the presence or absence of a suppressed function of a SWI/SNF complex factor in a cancer cell contained in a cancer patient-derived sample; and
(b) administering a GPX4 inhibitor to a cancer patient having the suppressed function of a SWI/SNF complex factor detected in the cancer cell (herein, sometimes referred to as a "cancer treatment method").

In the cancer cell growth inhibition method and the cancer treatment method of the present invention, the detection of a suppressed function of a SWI/SNF complex factor and the GPX4 inhibitor are as described above.

The suppression of growth of cancer cells includes acceleration of death of the cancer cells and suppression of growth of the cancer cells. The method for contacting a GPX4 inhibitor with a cancer cell is not particularly limited, and examples thereof include a method in which a GPX4 inhibitor is added to a culture medium for the cancer cell.

The amount of the GPX4 inhibitor to be contacted with the cancer cell may be an amount effective for inhibiting GPX4 to suppress growth of cancer cells, and is appropriately selected according to the properties of a compound inhibiting GPX4, the type of cancer cell, and the like.

The administration of the GPX4 inhibitor to a cancer patient may be oral administration or parenteral administration (e.g. intravenous administration, arterial administration or topical administration).

The dosage of the GPX4 inhibitor administered to a cancer patient may be an amount effective for treating cancer by inhibiting GPX4, and cannot be determined definitely because it is appropriately selected according to the properties of a compound inhibiting GPX4, the age, the body weight, the symptom and the physical condition of a cancer patient, the advancement state of cancer, and the like. For example, when the GPX4 inhibitor is administered to a human, the daily dosage thereof is 0.001 to 100,000 mg, preferably 0.01 to 5,000 mg, in terms of the amount of the compound inhibiting GPX4. The frequency of administration of the GPX4 inhibitor to a cancer patient also cannot be determined definitely, and it is preferable that for example, the GPX4 inhibitor be administered once or in two to four divided doses daily, with the administration being repeated at appropriate intervals. The dosage and the frequency of administration can be appropriately increased or decreased if necessary at a physician's discretion.

In this way, GPX4 is further inhibited in cancer cells having a suppressed function of a SWI/SNF complex factor in a cancer patient, so that the cancer can be treated by acceleration of death and/or suppression of growth of the cancer cells.

The cancer to be treated is not particularly limited, and include at least one selected from the group consisting of lung cancer, ovary cancer, uterus cancer, liver cancer, stomach cancer, esophagus cancer, bowel cancer, pancreas cancer, prostate cancer, bladder cancer and kidney cancer, and among them, at least one selected from the group consisting of lung cancer, ovary cancer, liver cancer, stomach cancer and pancreas cancer is preferable.

### <Reagent for detecting presence or absence of mutation>

The present invention also provides a reagent for detecting the presence or absence of a suppressed function of a SWI/SNF complex factor in the above-described method, the reagent comprising as an active ingredient the molecule of at least one of:
(i) an oligonucleotide primer binding specifically to a gene for a SWI/SNF complex factor;
(ii) an oligonucleotide probe binding specifically to a gene for a SWI/SNF complex factor; and
(iii) an antibody binding specifically to SWI/SNF complex factor protein.

The oligonucleotide primer may be designed on the basis of nucleotide sequence information of genome DNA and cDNA for a SWI/SNF complex factor (e.g. Tables 1 to 3) so as to ensure that the primer is consistent with the above-mentioned method and an amplification region, and production of amplified products of genes other than a gene for a SWI/SNF complex factor is avoided as much as possible. Those skilled in the art can design such an oligonucleotide primer by a conventional method. The oligonucleotide primer has a length of typically 15 to 50 bases, preferably 15 to 30 bases, and may have a larger or smaller length depending on a method and a purpose.

The oligonucleotide probe may be designed on the basis of nucleotide sequence information of genome DNA and cDNA for a SWI/SNF complex factor (e.g. Tables 1 to 3) so as to ensure that the probe is consistent with the above-mentioned method and a hybridization region, and hybridization to genes other than a gene for a SWI/SNF complex factor is avoided as much as possible. Those skilled in the art can design such an oligonucleotide probe by a conventional method. The oligonucleotide probe has a length of typically 15 to 200 bases, preferably 15 to 100 bases, still more preferably 15 to 50 bases, and may have a larger or smaller length depending on a method and a purpose.

It is preferable that the oligonucleotide probe be appropriately labeled and used. Examples of the method for performing labeling include a method in which using T4 polynucleotidekinase, the 5'-end of the oligonucleotide is phosphorylated with 32P to perform labeling; and a method in which using a DNA polymerase such as Klenow enzyme, a substrate base labeled with an isotope such as 32P, a fluorescent dye, biotin or the like with a random hexamer oligonucleotide or the like as a primer is incorporated (random priming method).

The oligonucleotide primer and the oligonucleotide probe can be prepared by, for example, a commercially available oligonucleotide synthesizing machine. It is also possible to prepare the oligonucleotide probe as a double-stranded DNA fragment obtained by restriction enzyme treatment or the like. The oligonucleotide primer and the oligonucleotide probe according to the present invention are not required to be composed only of a natural nucleotide (deoxyribonucleotide (DNA) or ribonucleotide (RNA)), and all or part thereof may be composed of a non-natural nucleotide. Examples of the non-natural nucleotide include PNA (polyamide nucleic acid), LNA (registered trademark, locked nucleic acid), ENA (registered trademark, 2'-O,4'-C-ethylene-bridged nucleic acids), and complexes thereof.

When the antibody binding specifically to the SWI/SNF complex factor protein is a polyclonal antibody, the antibody can be obtained by immunizing an immune animal with an antigen (e.g. SWI/SNF complex factor protein, a partial peptide thereof, or cells which express the protein or the peptide), and purifying antiserum of the animal by conventional means (e.g. salting-out, centrifugation, dialysis or column chromatography). The monoclonal antibody can be prepared by a hybridoma method or a recombinant DNA method.

Typical examples of the hybridoma method include a Kohler&Milstein method (Kohler&Milstein, Nature 1975; 256: 495). The antibody-producing cell used in a cell fusion step in this method is a spleen cell, a lymph node cell, a peripheral blood leukocyte or the like of an animal (e.g. mouse, rat, hamster, rabbit, monkey or goat) immunized with an antigen (e.g. SWI/SNF complex factor protein, a partial peptide thereof, or cells which express the protein or the peptide). It is also possible to use antibody-producing cells obtained by applying an antigen in a culture medium to the cells or lymphocytes isolated in advance from animals which are not immunized. As a myeloma cell, any of various known cell lines can be used. The antibody-producing cell and the myeloma cell may be derived from different animal species as long as these cells can be fused with each other, and cells derived from the same animal species are preferable. The hybridoma is produced by, for example, cell fusion between a spleen cell obtained from a mouse immunized with an antigen and a mouse myeloma cell, and by subsequent screening, a hybridoma which produces a monoclonal antibody specific to SWI/SNF complex factor protein can be obtained. The monoclonal antibody to SWI/SNF complex factor protein can be obtained by culturing the hybridoma or from ascites fluid of a mammal given the hybridoma.

The recombinant DNA method is a method in which DNA encoding the antibody is cloned from a hybridoma, a B cell or the like, and incorporated into an appropriate vector, and the vector is introduced into a host cell (e.g. mammal cell line, Bacillus coli, yeast cell, insect cell or plant cell) to produce an antibody according to the present invention as a recombinant antibody (e.g. P. J. Delves, Antibody Production: Essential Techniques, 1997 WILEY, P. Shepherd and C. Dean Monoclonal Antibodies, 2000 OXFORD UNIVERSITY PRESS, Vandamme AM et al., Eur. J. Biochem. 1990; 192: 767-775). In expression of DNA encoding an antibody, DNAs encoding a heavy chain or a light chain may be incorporated into different expression vectors to transform the host cell, or DNAs encoding a heavy chain and a light chain may be incorporated into a single expression vector to transform the host cell (e.g. WO 94/11523). The antibody can be obtained in a substantially pure and homogeneous form by culturing the host cell, separating the antibody from the inside of the host cell or the culture solution and purifying the antibody. For the separation and purification of the antibody, a common method which is used for purification of a polypeptide can be used. When a transgenic animal (e.g. bovine, goat, sheep or pig) into which an antibody gene is incorporated is prepared using a transgenic animal preparation technique, a large amount of a monoclonal antibody derived from the antibody gene can be obtained from milk from the transgenic animal.

On the basis of the thus-obtained antibodies or genes thereof, functional fractions of antibodies such as Fab, Fab', F(ab')₂, Fv, scFv, sc(Fv)₂, dsFv and diabodies, and multimers (e.g. dimers, trimers, tetramers and polymers) thereof can be prepared.

When the amount of an antibody bound to SWI/SNF complex factor protein is directly detected, the resulting anti-SWI/SNF complex factor protein antibody is directly labeled with an enzyme, a radioisotope, a fluorescent dye, an avidin-biotin system or the like and used. On the other hand, when an indirect detection method is carried out in which the amount of an antibody bound to SWI/SNF complex factor protein is detected using a secondary antibody etc., the resulting anti-SWI/SNF complex factor protein antibody (primary antibody) is not required to be labeled, and for the detection, a labeled molecule which recognizes the antibody (e.g. secondary antibody or protein A) may be used.

The reagent according to the present invention may comprise other ingredients acceptable for reagents, such as sterilized water, physiological saline, a buffering agent and a preservative if necessary in addition to the above-described molecule as an active ingredient.

### <Method for screening compounds to be used for treatment of cancer and cancer therapeutic drug>

The present invention provides:
a method for screening compounds to be used for treatment of cancer containing a cancer cell having a suppressed function of a SWI/SNF complex factor detected, the method comprising the step of:
selecting a compound on the basis of whether GPX4 is inhibited or not (hereinafter, sometimes referred to as a "method for screening compounds).

By using each of compounds which inhibit GPX4 and are screened by the method for screening compounds,
there can be provided a cancer therapeutic drug comprising a compound which inhibits GPX4 as an active ingredient, the cancer therapeutic drug being a therapeutic drug for cancer containing a cancer cell having a suppressed function of a SWI/SNF complex factor detected.

The test compound applied to the method for screening compounds according to the present invention is not particularly limited, and examples thereof include at least one selected from the group consisting of the above-described compound molecules listed as compounds which inhibit GPX4, polypeptides and polynucleotides. More specific examples of the test compound include synthetic low-molecular compound libraries, expressed products of gene libraries, peptide libraries, siRNA, antibodies, bacteria releasing substances, extracts and culture supernatants of cells (microorganisms, plant cells and animal cells), purified or partially purified polypeptides, marine organisms, plant or animal-derived extracts, and random phage peptide display libraries. The test compound may be a derivative of a known GPX4 inhibitor.

In the method for selecting a compound on the basis of whether GPX4 is inhibited or not (screening), a test compound may be applied to the system for confirmation of inhibition of activity or expression of GPX4, followed by detecting subsequent GPX4 activity or expression. When the result of detection shows that the activity or expression decreases as compared to GPX4 activity or expression in a control (e.g. a case where the test compound is not added), it can be evaluated that GPX4 is inhibited.

The "GPX4" which is evaluated for being inhibited or not by the compound in the screening is as described above.

The compound identified by the method for screening compounds according to the present invention can be formed into a cancer therapeutic drug as a medicament by appropriately mixing the compound with any of the pharmacologically acceptable additive ingredients mentioned for the GPX inhibitor above, etc. and subjecting the resulting mixture to formulation by a known pharmaceutical method. In particular, the compound can be formed into a therapeutic drug which is effective for cancer containing a cancer cell having a suppressed function of a SWI/SNF complex factor and is used for treating a cancer patient having a suppressed function of a SWI/SNF complex factor detected, and/or administered to the patient.

### [Example]

Hereinafter, the present invention will be described in more detail on the basis of Example, but the present invention is not limited to Examples below.

### 1. Experimental material and method

### (1) Cell line

The cancer cell lines shown in Table 4 above: NCI-H358, NCI-H2122, NCI-H2110, NCI-H1792, NCI-H1437, MOR, COV362, MKN-45, NCI-H838, B1203L (Int J Cancer. 2006; 118: 1992-7), SBC-5, TOV-21G, NCI-H1703, SNU-1327, NCI-H23, OVISE, NCI-H522 and SK-HEP-1 were used as cell lines to be tested in the present invention. The original sourced for the cell lines are shown in Table 5. As shown in Table 4 above, NCI-H23, NCI-H522, SBC-5, SK-HEP-1 and SNU-1327 are human cancer cell lines having a truncating mutation in a gene for SMARCA4, and NCI-H1703 is a human cancer cell line having a mutation at a splicing site of a gene for SMARCA4. NCI-H1703, NCI-H23, NCI-H522, SBC-5, SK-HEP-1 and NCI-H838 are human cancer cell lines in which expression of SMARCA4 protein is not detected in immunoblot analysis. NCI-H1703, NCI-H23, NCI-H522 and SBC-5 are human cancer cell lines in which SMARCA2 protein is not detected in immunoblot analysis. B1203L, OVISE and TOV-21G are human cancer cell lines having a truncating mutation in a gene for ARID1A, B1203L is a human cancer cell line having a truncating mutation in a gene for ARID2, OVISE is a human cancer cell line having a truncating mutation in a gene for ARID1B, and NCI-H838 is a human cancer cell line having a truncating mutation in a gene for BCL11B. On the other hand, NCI-H358, NCI-H2122, NCI-H2110, NCI-H1792, NCI-H1437, MOR, COV362 and MKN-45 are human cancer cell lines for which information about a loss-of-function mutation of a gene for a SWI/SNF complex factor and expression of protein has not been published.

### (2) Small interfering (si)RNA

For suppression of expression of GPX4, ON-TARGETplus Individual siRNA (manufactured by Dharmacon, Inc.) was used. For transfection, Lipofectamine RNAiMAX (manufactured by Thermo Fisher Scientific, product code: 13778150) was used. Non-target siRNA (ON-TARGET plus Non-targeting Control, Dharmacon, Inc. product code: D-001810-01, SEQ ID NO: 60) was used as a negative control, and PLK1 (Dharmacon, Inc. product code: J-003290-09, SEQ ID NO: 59) was used as a positive control. For dilution of each siRNA, the siRNA was dissolved using a 1 × siRNA buffer obtained by diluting a 5 × siRNA buffer (manufactured by Dharmacon: B-002000-UB-100) by 5 times with nuclease free water (AM 9932 manufactured by Ambion, Inc.).

### (3) Test for suppression of expression of GPX4

In the cancer cell lines described in (1) above and shown in Table 4, cell growth inhibition activity under suppression of expression of GPX4 was evaluated. Each cell line was seeded at 100 µL/well on a 96-well plate (manufactured by Greiner Bio-One GmbH) so as to attain the seeding density (1) or (2) shown in Table 5 below. For transfection evaluation, GPX4 siRNA (#1, #2 and #3), a negative control (ON-TARGET plus Non-targeting Control) and a positive control (PLK1 siRNA) shown in Table 6 below were used. The final concentration of each siRNA was 0.1 to 10 nM, and for transfection, Lipofectamine RNAiMAX (manufactured by Thermo Fisher Scientific, product code: 13778150) was used. The cells were cultured for 7 days, and the intracellular ATP which is a marker for cell survival was then measured using Cell Titer-Glo 2.0 Cell Viability Assay (manufactured by Promega Corporation). The cell growth inhibition ratio in suppression of expression of GPX4 was calculated, where the value for the cells transfected with the negative control and cultured for 7 days and the value for the cells transfected with the positive control and cultured for 7 days were defined as 0% and 100%, respectively.

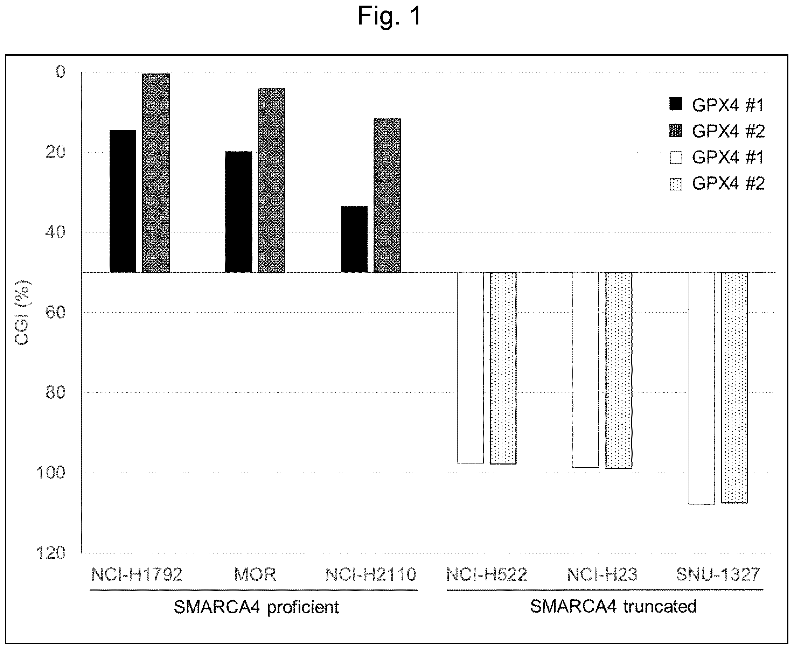

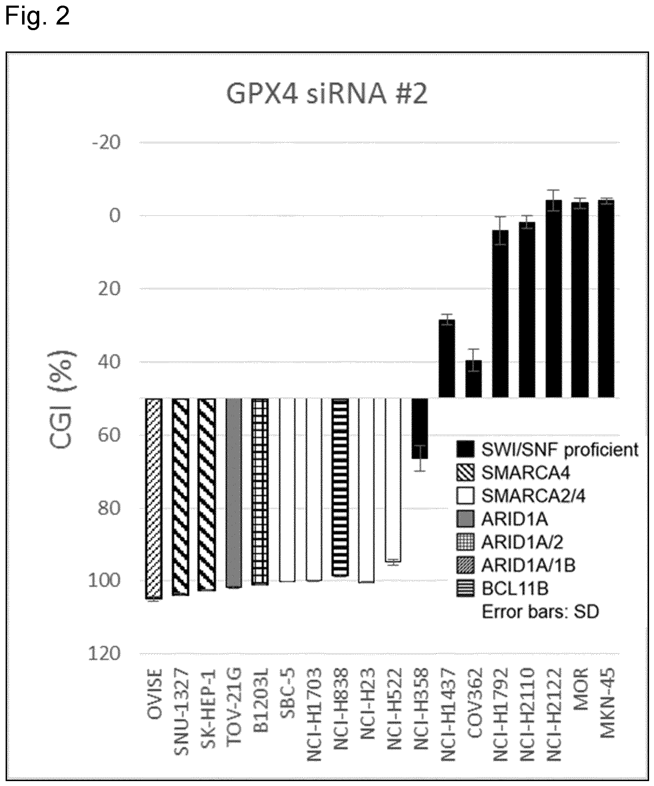

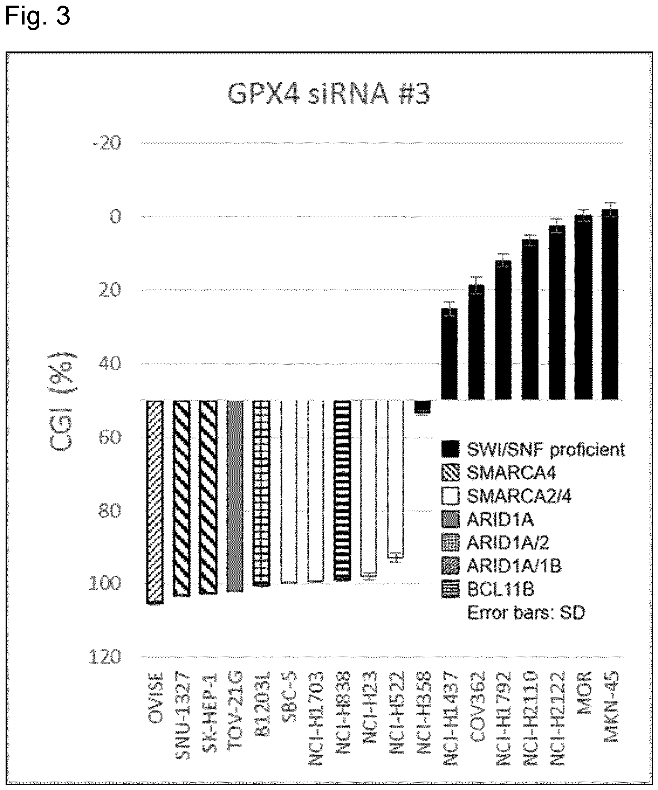

Figure 1 shows cell growth inhibition ratios (CGI (%)) in suppression of expression of GPX4 by GPX4 siRNA #1 or #2 in the cell lines (lung cancer cell lines) of NCI-H1792, MOR, NCI-H2110, NCI-H522, NCI-H23 and SNU-1327. Figure 2 shows cell growth inhibition ratios (CGI (%)) in suppression of expression of GPX4 by GPX4 siRNA #2 and Figure 3 shows cell growth inhibition ratios (CGI (%)) in suppression of expression of GPX4 by GPX4 siRNA #3 in the cell lines (including lung cancer cell lines, ovary cancer cell lines, liver cancer cell lines and stomach cancer cell lines) shown in Table 4 and tested at a time different from that for Figure 1.

### (4) Test for inhibition of activity of GPX4 1

In each cancer cell line, cell growth inhibition activity under a GPX4 inhibitor was evaluated. As the GPX4 inhibitor, ML210 (CAS No: 1360705-96-9) which is a low-molecular GPX4 inhibitor reported to be a compound inhibiting the enzymatic activity of GPX4: 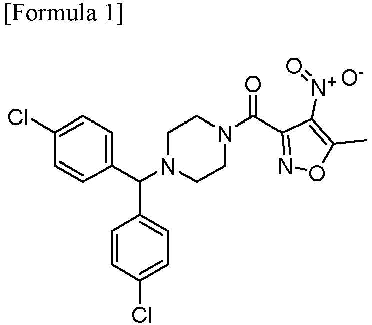 or RSL3 (CAS No: 1219810-16-8): 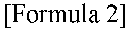 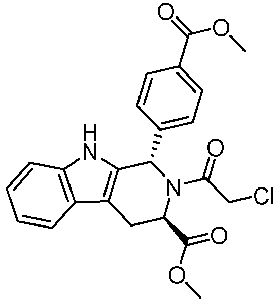 was used.

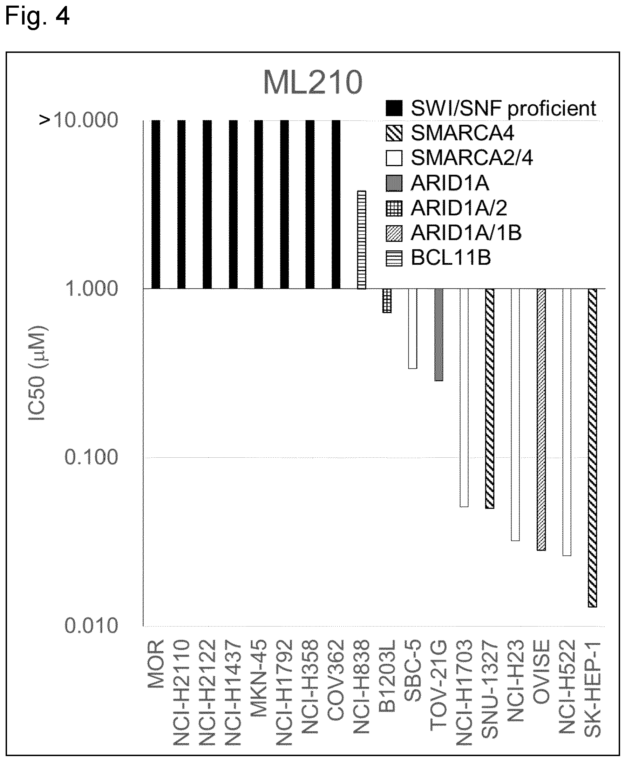

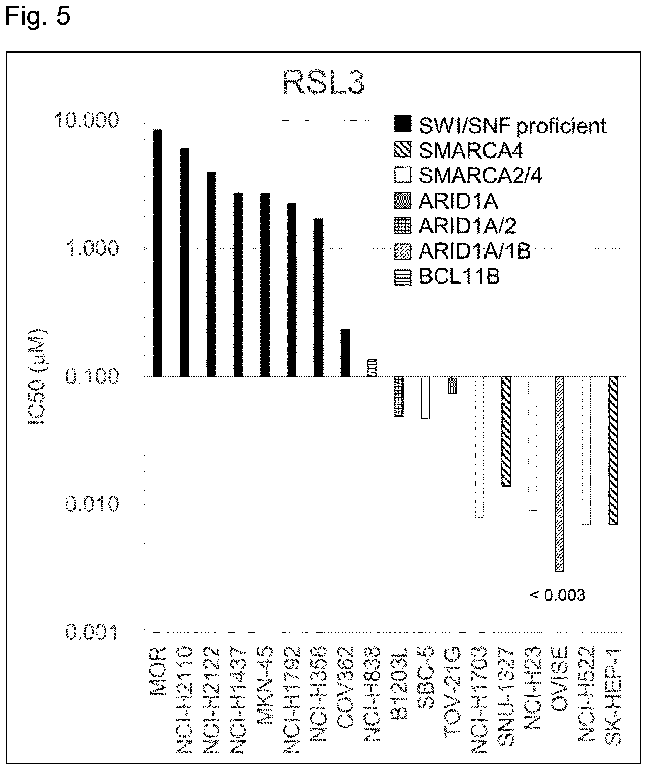

These compounds were each added, and each cell line was seeded at 100 µL/well so as to attain the seeding density (2) shown in Table 5 below. The cells were cultured for 7 days, the intracellular ATP which is a marker for cell survival was then measured using Cell Titer-Glo 2.0 Cell Viability Assay (manufactured by Promega Corporation), and the cell survival rate was calculated, where the intracellular ATP level in each cancer cell line given only a solvent (dimethyl sulfoxide (DMSO) or water) for the compound and cultured for 7 days was defined as 100%. IC50 (50% inhibition concentration (µM) was calculated from a cell survival curve obtained from the concentration of each compound and the cell survival rate. Figure 4 shows IC50 (µM) in the cell lines when ML210 was used as the GPX4 inhibitor, and Figure 5 shows IC50 (µM) in the cell lines when RSL3 was used as the GPX4 inhibitor.

### (5) Test for suppression of expression of GPX family protein

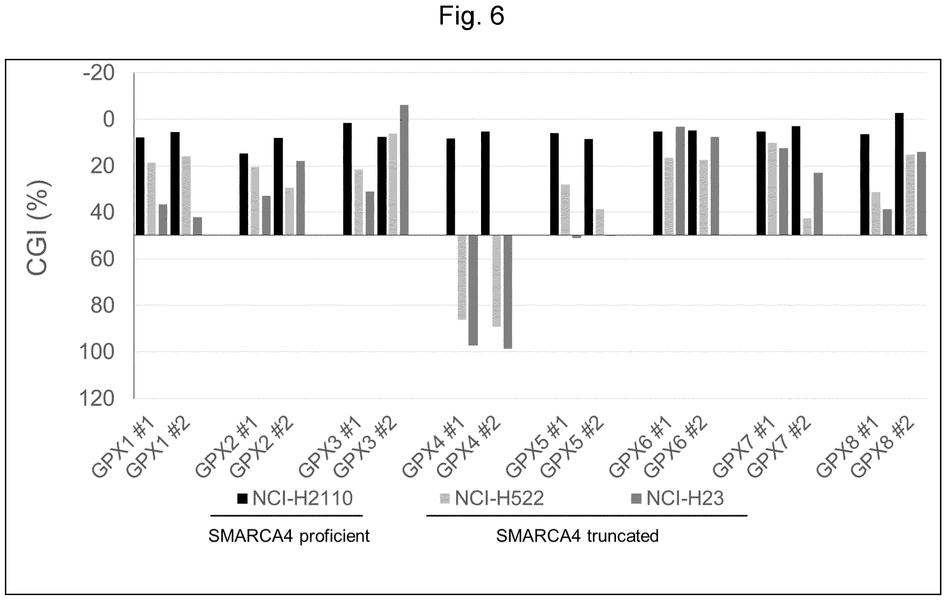

In the cancer cell lines, cell growth inhibition activity under suppression of expression of GPX1 to GPX8 which are GPX family proteins was evaluated. Each cell line was seeded at 100 µL/well on a 96-well plate so as to attain the seeding density (2) shown in Table 5 below. For transfection evaluation, GPX1 siRNA (#1 and #2), GPX2 siRNA (#1 and #2), GPX3 siRNA (#1 and #2), GPX4 siRNA (#1 and #2), GPX5 siRNA (#1 and #2), GPX6 siRNA (#1 and #2), GPX7 siRNA (#1 and #2) and GPX8 siRNA (#1 and #2) (all from Dharmacon, Inc.) shown in Table 6 below were used, respectively. The final concentration of each siRNA was 5 nM or 10 nM, and for transfection, Lipofectamine RNAiMAX (Thermo Fisher Scientific, product code: 13778150) was used. The cells were cultured for 7 days, and the intracellular ATP which is a marker for cell survival was then measured using Cell Titer-Glo 2.0 Cell Viability Assay (manufactured by Promega Corporation). The cell growth inhibition ratio in suppression of expression of each GPX family protein was calculated, where the value for the cells transfected with the negative control and cultured for 7 days and the value for the cells transfected with the positive control and cultured for 7 days were defined as 0% and 100%, respectively. Figure 6 shows cell growth inhibition ratios (CGI (%)) in suppression of expression of GPX family proteins in the cell lines of NCI-H2110, NCI-H522 and NCI-H23.

### (6) Test for suppression of expression of protein involved in glutathione synthesis

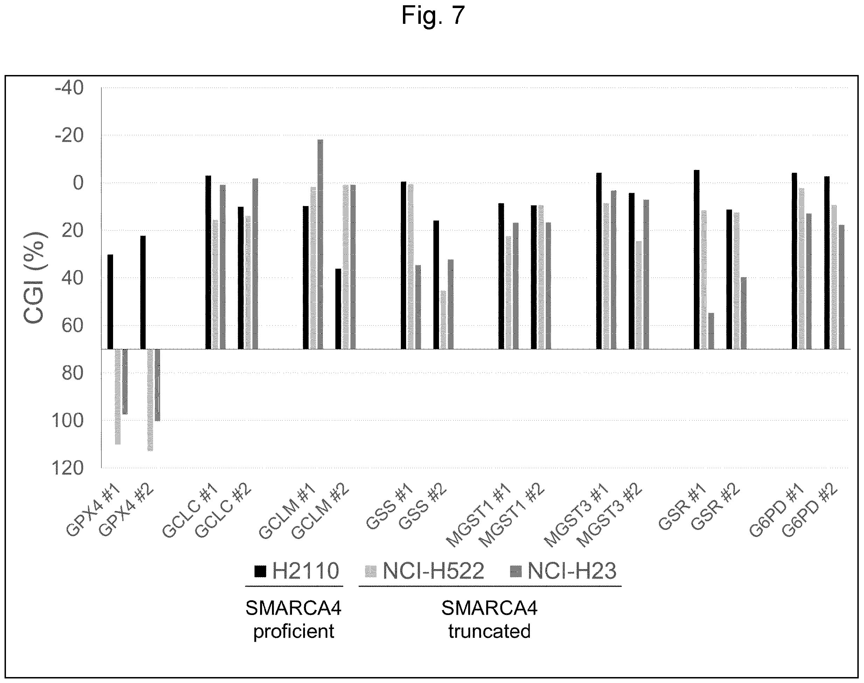

In the cancer cell lines, cell growth inhibitory activity under suppression of expression of protein which is involved in glutathione synthesis (protein involved in glutathione synthesis) was evaluated. Each cell line was seeded at 100 µL/well on a 96-well plate so as to attain the seeding density (2) shown in Table 5 below. For the transfection assay, GPX4 siRNA (#1 and #2), GCLC siRNA (#1 and #2), GCLM siRNA (#1 and #2), GSS siRNA (#1 and #2), MGST1 siRNA (#1 and #2), MGST3 siRNA (#1 and #2), GSR siRNA (#1 and #2) and G6PD siRNA (#1 and #2) (all from Dharmacon, Inc.) shown in Table 6 below were used, respectively. The final concentration of each siRNA was 5 nM, and for transfection, Lipofectamine RNAiMAX (Thermo Fisher Scientific, product code: 13778150) was used. The cells were cultured for 7 days, and the intracellular ATP which is a marker for cell survival was then measured using Cell Titer-Glo 2.0 Cell Viability Assay (manufactured by Promega Corporation). The cell growth inhibition ratio in suppression of expression of each protein involved in glutathione synthesis was calculated, where the value for the cells transfected with the negative control and cultured for 7 days and the value for the cells transfected with the positive control and cultured for 7 days were defined as 0% and 100%, respectively. Figure 7 shows cell growth inhibition ratios (CGI (%)) in suppression of expression of proteins involved in glutathione synthesis in the cell lines of NCI-H2110, NCI-H522 and NCI-H23.

**[Table 5]**

| Cell line | Culture solution | Seeding density (cells/well) (1) | Seeding density (cells/well) (2) | Original source |
|---|---|---|---|---|
| B1203L | RPMI-1640, 10% FBS | 2000 | 2000 | RIKEN Cell Bank* |
| COV362 | D-MEM(high glucose), 10% FBS | 2000 | 1000 | ECACC 07071910 |
| MKN-45 | RPMI-1640, 10% FBS | 3000 | 1500 | JCRB Cell Bank |
| MOR | RPMI-640, 10% FBS | 5000 | 3000 | ECACC 84112312 |
| NCI-H1437 | RPMI-640, 10% FBS | 1500 | 2000 | ATCC |
| NCI-H1703 | RPMI-1640, 10% FBS, 10mM HEPES, 0.45% D-glucose, 1mM Sodium Pyruvate | 1500 | 3000 | ATCC |
| NCI-H1792 | RPMI-1640, 10% FBS, 10mM HEPES, 0.45% D-glucose, 1mM Sodium Pyruvate | 3000 | 5000 | ATCC |
| NCI-H2110 | RPMI-1640, 10% FBS, 10mM HEPES, 0.45% D-glucose, 1mM Sodium Pyruvate | 5000 | 5000 | ATCC |
| NCI-H2122 | RPMI-1640, 10% FBS, 10mM HEPES, 0.45% D-glucose, 1mM Sodium Pyruvate | 3000 | 3000 | ATCC |
| NCI-H23 | RPMI-1640, 10% FBS, 10mM HEPES, 0.45% D-glucose, 1mM Sodium Pyruvate, 2mM L-glutamine | 1500 | 1500 or 2000 | ATCC |
| NCI-H358 | RPMI-1640, 10% FBS | 3000 | 3000 | ATCC |
| NCI-H522 | RPMI-1640, 10% FBS, 10mM HEPES, 0.45% D-glucose, 1mM Sodium Pyruvate, 2mM L-glutamine | 5000 | 2000 | ATCC |
| NCI-H838 | RPMI-1640, 10% FBS, 10mM HEPES, 0.45% D-glucose, 1mM Sodium Pyruvate | 500 | 1000 | ATCC |
| OVISE | RPMI-640, 10% FBS | 4000 | 2000 | JCRB Cell Bank |
| SBC-5 | E-MEM, 10% FBS, 1% NEAA, 1mM Sodium Pyruvate | 4000 | 4000 | JCRB Cell Bank |
| SK-HEP-1 | D-MEM(high glucose), 10% FBS | 2000 | 3000 | ATCC |
| SNU-1327 | RPMI-1640, 10% FBS, 25 mM HEPES | 5000 | 3000 | KCLB |
| TOV21G | MCDB105:medium199=1:1, 15% FBS, | 1000 | 2000 | ATCC |

| | | | | |
|---|---|---|---|---|
| ECACC: European Collection of Authenticated Cell Cultures ATCC: American Type Culture Collection KCLB: Korean Cell Line Bank * B1203L was provided by the RIKEN BRC through the National Bio-Resource Project of the MEXT, Japan. | | | | |

**[Table 6]**

| Duplex Catalogue No. | siRNA | GENE ID | NCBI Reference ID | GI Number | SEQ ID NO: |
|---|---|---|---|---|---|
| J-003290-09 | PLK1 | 5347 | NM_005030 | 34147632 | 59 |
| D-001810-01 | ON-TARGETplus Non-targeti ng Control | - | - | - | 60 |
| J-008982-05 | GPX1 siRNA #1 | 2876 | NM_000581 | 41406083 | 61 |
| J-008982-06 | GPX1 siRNA #2 | 2876 | NM_000581 | 41406083 | 62 |
| J-011675-06 | GPX2 siRNA #1 | 2877 | NM_002083 | 32967606 | 63 |
| J-011675-07 | GPX2 siRNA #2 | 2877 | NM_002083 | 32967606 | 64 |
| J-006485-06 | GPX3 siRNA #1 | 2878 | NM_002084 | 6006000 | 65 |
| J-006485-07 | GPX3 siRNA #2 | 2878 | NM_002084 | 6006000 | 66 |
| J-011676-06 | GPX4 siRNA #1 | 2879 | NM_002085 | 75709199 | 67 |
| J-011676-07 | GPX4 siRNA #2 | 2879 | NM_002085 | 75709199 | 68 |
| J-009445-20 | GPX5 siRNA #1 | 2880 | NM_003996 | 209977068 | 69 |
| J-009445-21 | GPX5 siRNA #2 | 2880 | NM 003996 | 209977068 | 70 |
| J-019309-10 | GPX6 siRNA #1 | 257202 | NM_182701 | 33186886 | 71 |
| J-019309-11 | GPX6 siRNA #2 | 257202 | NM_182701 | 33186886 | 72 |
| J-009875-09 | GPX7 siRNA #1 | 2882 | NM_015696 | 15618996 | 73 |
| J-009875-11 | GPX7 siRNA #2 | 2882 | NM_015696 | 15618996 | 74 |
| J-034902-07 | GPX8 siRNA #1 | 493869 | NM_001008397 | 56605999 | 75 |
| J-034902-08 | GPX8 siRNA #2 | 493869 | NM_001008397 | 56605999 | 76 |
| J-009212-09 | GCLC siRNA #1 | 2729 | NM_001498 | 45359851 | 77 |
| J-009212-10 | GCLC siRNA #2 | 2729 | NM_001498 | 45359851 | 78 |
| J-011670-09 | GCLM siRNA #1 | 2730 | NM_002061 | 53759142 | 79 |
| J-011670-10 | GCLM siRNA #2 | 2730 | NM_002061 | 53759142 | 80 |
| J-009586-05 | GSS siRNA #1 | 2937 | NM_000178 | 30581166 | 81 |
| J-009586-07 | GSS siRNA #2 | 2937 | NM_000178 | 30581166 | 82 |
| J-009248-05 | MGST1 siRNA #1 | 4257 | NM_145792 | 22035637 | 83 |
| J-009248-06 | MGST1 siRNA #2 | 4257 | NM_145792 | 22035637 | 84 |
| J-008382-09 | MGST3 siRNA #1 | 4259 | NM_004528 | 22035640 | 85 |
| J-008382-10 | MGST3 siRNA #2 | 4259 | NM_004528 | 22035640 | 86 |
| J-009647-07 | GSR siRNA #1 | 2936 | NM_000637 | 50301237 | 87 |
| J-009647-08 | GSR siRNA #2 | 2936 | NM_000637 | 50301237 | 88 |
| J-008181-18 | G6PD siRNA #1 | 2539 | NM_001042351 | 108773792 | 89 |
| J-008181-19 | G6PD siRNA #2 | 2539 | NM_001042351 | 108773792 | 90 |
| J-011676-05 | GPX4 siRNA #3 | 2879 | NM_002085 | 75709199 | 91 |

Table 5 shows seeding densities of the cell lines in the tests and culture solutions and original sources for the cell lines. Table 6 shows sequence numbers showing the sequences of the siRNAs, and IDs (gene IDs) in the NCBI RefSeq database, reference IDs (NCBI reference IDs) and GI numbers for genes whose expression is suppressed by the siRNAs.

### (7) Test for inhibition of activity of GPX4 2 (comparative test with GCLC inhibitor)

In each cancer cell line, cell growth inhibition activity under a GPX4 inhibitor was evaluated. As the GPX4 inhibitor, the ML210 or RSL3 was used. As a comparative control, buthionine sulphoximine (BSO, CAS No: 83730-53-4) that is an inhibitor of protein GCLC which is involved in glutathione synthesis (catalyst subunit of glutamate-cysteine ligase (GCL) that is a rate limiting enzyme for glutathione synthesis): 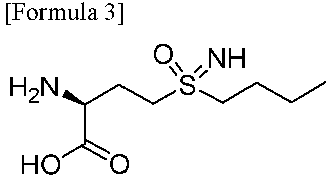 was used. These compounds were each added, and each cell line was seeded at 100 µL/well so as to attain the seeding density (2) shown in Table 5 above. The cells were cultured for 7 days, the intracellular ATP which is a marker for cell survival was then measured using Cell Titer-Glo 2.0 Cell Viability Assay (manufactured by Promega Corporation), and the cell survival rate was calculated, where the intracellular ATP level in each cancer cell line given only a solvent (dimethyl sulfoxide (DMSO) or water) for the compound and cultured for 7 days was defined as 100%. IC50 (50% inhibition concentration (µM) was calculated from a cell survival curve obtained from the concentration of each compound and the cell survival rate.

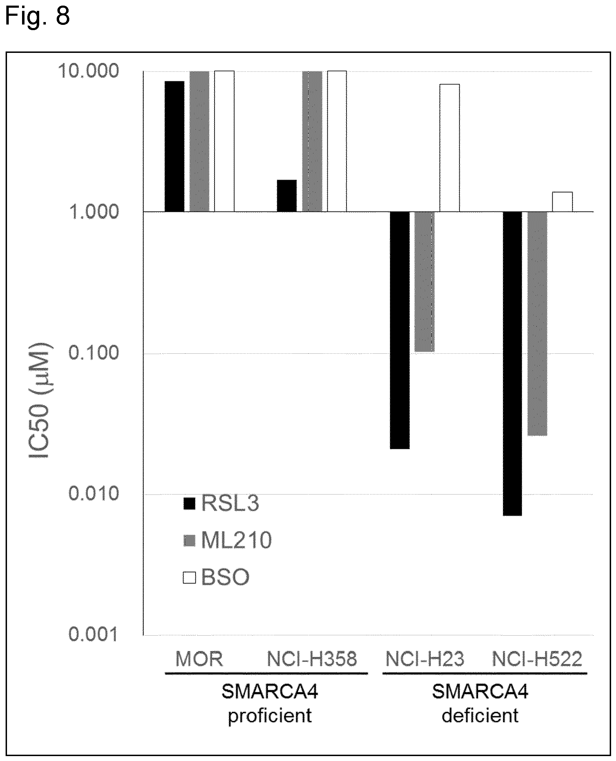

Figure 8 shows IC50 (µM) in the cell lines MOR, NCI-H 358, NCI-H23 and NCI-H522 when ML210, RSL3 or BSO is used.

### (8) Test for drug efficacy of ML210 on SK-HEP-1 tumor-bearing mouse

For examining the efficacy of the GPX4 inhibitor on SWI/SNF complex factor-deficient cancer *in vivo,* a test was conducted in which ML210 was administered to mice in which SK-HEP-1 (human cancer cell line suppressing a function of SMARCA4) was transplanted (SK-HEP-1 tumor-bearing mice). Specifically, first, SK-HEP-1 was cultured *in vitro,* and collected using a solution at 2.5 g/L-tripsin/1 mmol-EDTA (manufactured by Nacalai tesque, product code: 35554-64) on the day of inoculation to the mice, and the cells were suspended at 2.5 E7 cells/mL in a solution of Matrigel (manufactured by Corning Incorporated, product name: 356234) diluted with the Hanks' Balanced Salt solution (manufactured by Sigma-Aldrich Co. LLC, product code: H 9269) (final concentration: 50%). 0.2 mL of the prepared cell suspension was transplanted into the groin of each of BALB/c-nu/nu mice (CAnN. Cg-Foxn 1 <nu>/CrlCrlj, Charles River, Inc.).

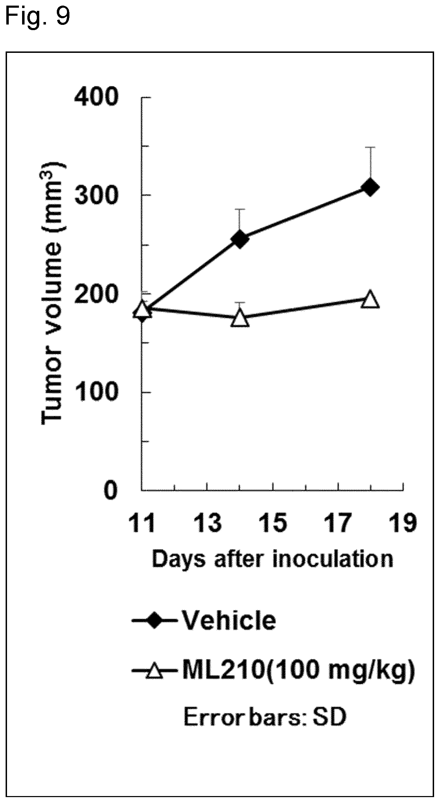

Subsequently, to mice (SK-HEP-1 tumor-bearing mice) for which the engraftment of the tumor (cancer cell) had been confirmed, a dosing liquid obtained by suspending ML210 at a predetermined concentration in a mixed liquid of 10% dimethyl sulfoxide (manufactured by Wako Company, product code: 043-07216), 10% Cremophor (manufactured by Sigma-Aldrich Co. LLC, product code: C5135), 15% polyethylene glycol 400 (manufactured by Wako Company, product code: 161-09065) and 15% hydroxypropyl-β-cyclodextrin (manufactured by Nihon Shokuhin Kako Co., Ltd., product code: 7585-39-9) was orally administered at a predetermined dose (20 mL/kg) once a day to a daily dosage of 100 mg/kg upon the confirmation of the implantation. The day of inoculation of SK-HEP-1 was defined as day 0, administration of ML210 was started on day 11, and the volume of the tumor was measured on days 11, 14 and 18. The minor diameter and the major diameter of the tumor were measured using an electronic caliper (manufactured by Mitutoyo Corporation, product code: CD-15AX), and the volume of the tumor was calculated by the least square method. For SK-HEP-1 tumor-bearing mice given only the mixed liquid (vehicle) which does not contain ML210, similarly the day of inoculation of SK-HEP-1 was defined as day 0, administration of the vehicle was started on day 11, and the volume of the tumor was measured on days 11, 14 and 18. Figure 9 shows a relationship between the volume of the tumor in the group of SK-HEP-1 tumor-bearing mice given ML210 (ML210) or the group of SK-HEP-1 tumor-bearing mice given the mixed liquid (Vehicle: without administration of ML210) (Tumor Volume) and the time after inoculation of SK-HEP-1 (Days after inoculation).

### 2. Results

### (1) Test for suppression of expression of GPX4

In cancer cell lines having a deficient mutation in a gene for SMARCA4 that is a SWI/SNF complex factor and/or having no SMARCA4 protein detected by immunoblot analysis, cell growth was markedly inhibited to the extent that the cell growth inhibition ratio was 90% or more in the test for suppression of expression of GPX4 as shown in Figure 1. Further, even in cancer cell lines having a deficient mutation in a gene for at least one of SWI/SNF complex factors in addition to SMARCA4 (e.g. SMARCA2, ARID1A, ARID1B, ARID2 and BCL11B) and/or having at least one of these factors whose expression was not detected by immunoblot analysis, cell growth was markedly inhibited to the extent that the cell growth inhibition ratio was 90% or more when expression of GPX 4 was suppressed as shown in Figures 2 and 3. These results show that survival of a cancer cell line having a suppressed function of a SWI/SNF complex factor heavily depends on the function of GPX4.

### (2) Test for inhibition of activity of GPX4 1

When a GPX4 inhibitor was added to cancer cell lines having a deficient mutation in a gene for at least one of SWI/SNF complex factors (e.g. SMARCA4, SMARCA2, ARID1A, ARID1B, ARID2 and BCL11B) and/or having at least one of these factors whose expression was not detected by immunoblot analysis, cell growth was markedly inhibited as compared to cell lines having no suppressed function of a SWI/SNF complex factor in the test for inhibition of activity of GPX4 1 as shown in Figures 4 and 5.

The results in (1) and (2) above show that inhibition (suppression of expression and inhibition of activity) of GPX4 is effective for suppressing growth of cancer cells having a suppressed function of a SWI/SNF complex factor (obtaining antitumor activity).

### (3) Test for suppression of expression of GPX family protein

In cancer cell lines having a deficient mutation in a gene for SMARCA4 that is a SWI/SNF complex factor and/or having no SMARCA4 protein detected by immunoblot analysis, cell growth was markedly inhibited only when expression of GPX4, in particular, among GPX family proteins, was suppressed in the test for suppression of expression of GPX family protein as shown in Figure 6. This result shows that inhibition of GPX4 is important for obtaining antitumor activity against a cancer cell having a suppressed function of a SWI/SNF complex factor (e.g. SMARCA4).

### (4) Test for suppression of expression of protein involved in glutathione synthesis

In cancer cell lines having a deficient mutation in a gene for SMARCA4 that is a SWI/SNF complex factor and/or having no SMARCA4 protein detected by immunoblot analysis, cell growth was markedly inhibited only when expression of GPX4, in particular, among proteins involved in glutathione synthesis, was suppressed in the test for suppression of expression of protein involved in glutathione synthesis as shown in Figure 7. This result also shows that inhibition of GPX4 is important for obtaining antitumor activity against a cancer cell having a suppressed function of a SWI/SNF complex factor (e.g. SMARCA4).

### (5) Test for inhibition of activity of GPX4 2 (comparative test with GCLC inhibitor)

When a GPX4 inhibitor was added to cancer cell lines having a deficient mutation in a gene for SMARCA4 that is a SWI/SNF complex factor and/or having no SMARCA4 protein detected by immunoblot analysis, cell growth was markedly inhibited as compared to BSO which is a GCLC inhibitor in the test for inhibition of activity of GPX4 2 as shown in Figure 8. This result also shows that inhibition of GPX4 is effective for obtaining antitumor activity against a cancer cell having a suppressed function of a SWI/SNF complex factor (e.g. SMARCA4).

### (6) Test for drug efficacy of ML210 on SK-HEP-1 tumor-bearing mouse

As shown in Figure 9, the GPX4 inhibitor exhibited antitumor activity even *in vivo* against cancer (tumor) containing a cancer cell (cancer cell suppressing a function of SMARCA4) having a deficient mutation in a gene for SMARCA4 that is a SWI/SNF complex factor and/or having no SMARCA4 protein detected by immunoblot analysis. Specifically, in the group of SK-HEP-1 tumor-bearing mice given ML210, an increase in volume of the tumor was suppressed as compared to the group given the vehicle, and marked antitumor activity was observed.

### [Industrial Applicability]

As described above, according to the present invention, it is possible to efficiently predict sensitivity to cancer treatment with a GPX4 inhibitor using a suppressed function of a SWI/SNF complex factor as an indicator. In addition, according to the present invention, it is possible to detect the presence or absence of a suppressed function of a SWI/SNF complex factor in a cancer patient-derived sample and select a patient having the mutation detected, followed by subjecting the patient to treatment of cancer with a GPX4 inhibitor. This enables significant improvement of cancer treatment outcomes. By using a probe or a primer against a gene for a SWI/SNF complex factor and an antibody against a SWI/SNF complex factor, companion diagnosis can be efficiently performed by detection of the presence or absence of such a suppressed function of the SWI/SNF complex factor.

### [Sequence listing free text]

SEQ ID NO: 2
   <223> Xaa represents selenocysteine
SEQ ID NO: 59
   <223> PLK1
SEQ ID NO: 60
   <223> ON-TARGETplus Non-targeting Control
SEQ ID NO: 61
   <223> GPX1 siRNA #1
SEQ ID NO: 62
   <223> GPX1 siRNA #2
SEQ ID NO: 63
   <223> GPX2 siRNA #1
SEQ ID NO: 64
   <223> GPX2 siRNA #2
SEQ ID NO: 65
   <223> GPX3 siRNA #1
SEQ ID NO: 66
   <223> GPX3 siRNA #2
SEQ ID NO: 67
   <223> GPX4 siRNA #1
SEQ ID NO: 68
   <223> GPX4 siRNA #2
SEQ ID NO: 69
   <223> GPX5 siRNA #1
SEQ ID NO: 70
   <223> GPX5 siRNA #2
SEQ ID NO: 71
   <223> GPX6 siRNA #1
SEQ ID NO: 72
   <223> GPX6 siRNA #2
SEQ ID NO: 73
   <223> GPX7 siRNA #1
SEQ ID NO: 74
   <223> GPX7 siRNA #2
SEQ ID NO: 75
   <223> GPX8 siRNA #1
SEQ ID NO: 76
   <223> GPX8 siRNA #2
SEQ ID NO: 77
   <223> GCLC siRNA #1
SEQ ID NO: 78
   <223> GCLC siRNA #2
SEQ ID NO: 79
   <223> GCLM siRNA #1
SEQ ID NO. 80
   <223> GCLM siRNA #2
SEQ ID NO: 81
   <223> GSS siRNA #1
SEQ ID NO: 82
   <223> GSS siRNA #2
SEQ ID NO: 83
   <223> MGST1 siRNA #1
SEQ ID NO: 84
   <223> MGST1 siRNA #2
SEQ ID NO: 85
   <223> MGST3 siRNA #1
SEQ ID NO: 86
   <223> MGST3 siRNA #2
SEQ ID NO: 87
   <223> GSR siRNA #1
SEQ ID NO: 88
   <223> GSR siRNA #2
SEQ ID NO: 89
   <223> G6PD siRNA #1
SEQ ID NO: 90
   <223> G6PD siRNA #2
SEQ ID NO: 91
   <223> GPX4 siRNA #3

## Claims

1. A cancer therapeutic drug comprising a compound which inhibits GPX4 as an active ingredient, the cancer therapeutic drug being a therapeutic drug for cancer containing a cancer cell having a suppressed function of a SWI/SNF complex factor detected.

2. A cancer therapeutic drug comprising a compound which inhibits GPX4 as an active ingredient, the cancer therapeutic drug being a therapeutic drug for treating a cancer patient having a suppressed function of a SWI/SNF complex factor detected in a cancer cell contained in a cancer patient-derived sample.

3. A cancer therapeutic drug comprising a compound which inhibits GPX4 as an active ingredient, the cancer therapeutic drug being a therapeutic drug to be administered to a cancer patient having a suppressed function of a SWI/SNF complex factor detected in a cancer cell contained in a cancer patient-derived sample.

4. A method for predicting sensitivity of a cancer cell to a GPX4 inhibitor, the method comprising the step of:
predicting a cancer cell having a suppressed function of a SWI/SNF complex factor detected in the cancer cell, as having sensitivity to a GPX4 inhibitor.

5. A method for predicting sensitivity of a cancer patient to treatment with a GPX4 inhibitor, the method comprising the step of:
predicting a cancer patient having a suppressed function of a SWI/SNF complex factor detected in a cancer cell contained in a cancer patient-derived sample, as having sensitivity to treatment with a GPX4 inhibitor.

6. A method for selecting a cancer patient for cancer treatment with a GPX4 inhibitor, the method comprising the step of:
selecting a cancer patient having a suppressed function of a SWI/SNF complex factor detected in a cancer cell contained in a cancer patient-derived sample, for cancer treatment with a GPX4 inhibitor.

7. A method for suppressing growth of cancer cells having a suppressed function of a SWI/SNF complex factor detected, the method comprising the step of:
contacting a GPX4 inhibitor with a cancer cell having the suppressed function of a SWI/SNF complex factor detected.

8. A method for treating cancer, the method comprising the step of:
administering a GPX4 inhibitor to a cancer patient having a suppressed function of a SWI/SNF complex factor detected in a cancer cell contained in a cancer patient-derived sample.

9. A method for screening compounds to be used for treatment of cancer containing a cancer cell having a suppressed function of a SWI/SNF complex factor detected, the method comprising the step of:
selecting a compound on the basis of whether GPX4 is inhibited or not.

10. The cancer therapeutic drug according to any one of claims 1 to 3, wherein the SWI/SNF complex factor is a BAF complex factor.

11. The method according to any one of claims 4 to 9, wherein the SWI/SNF complex factor is a BAF complex factor.

12. The cancer therapeutic drug according to any one of claims 1 to 3, wherein the SWI/SNF complex factor is at least one selected from the group consisting of SMARCA2, SMARCA4, ARID1A, ARID1B, ARID2 and BCL11B.

13. The method according to any one of claims 4 to 9, wherein the SWI/SNF complex factor is at least one selected from the group consisting of SMARCA2, SMARCA4, ARID1A, ARID1B, ARID2 and BCL11B.

14. The cancer therapeutic drug according to any one of claims 1 to 3, wherein the suppressed function of a SWI/SNF complex factor is a decrease in activity of a SWI/SNF complex having the SWI/SNF complex factor as a constituent factor and/or a decrease in expression of the SWI/SNF complex factor.

15. The method according to any one of claims 4 to 9, wherein the suppressed function of a SWI/SNF complex factor is a decrease in activity of a SWI/SNF complex having the SWI/SNF complex factor as a constituent factor and/or a decrease in expression of the SWI/SNF complex factor.

16. The cancer therapeutic drug according to any one of claims 1 to 3, wherein the suppressed function of a SWI/SNF complex factor is a loss-of-function mutation of a gene for the SWI/SNF complex factor.

17. The method according to any one of claims 4 to 9, wherein the suppressed function of a SWI/SNF complex factor is a loss-of-function mutation of a gene for the SWI/SNF complex factor.
